# EUROPEAN PATENT SPECIFICATION

(11) **EP 4 501 259 B1**
(45) Date of publication and mention of the grant of the patent: **25.11.2026**
(21) Application number: 24221911.1
(22) Date of filing: 05.05.2021
(51) Int. Cl.: A61B 17/22, A61B 17/3207

(54) **ENDOVASCULAR DEVICE FOR CROSSING THROUGH AN OBSTRUCTION IN A BLOOD VESSEL**
ENDOVASKULÄRE VORRICHTUNG ZUM DURCHQUEREN EINER VERSTOPFUNG IN EINEM BLUTGEFÄSS
DISPOSITIF ENDOVASCULAIRE PERMETTANT DE FRANCHIR UNE OBSTRUCTION DANS UN VAISSEAU SANGUIN

(30) Priority: 05.05.2020 GB 202006665; 06.11.2020 WO PCT/EP2020/081386; 06.11.2020 WO PCT/EP2020/081399
(43) Date of publication of application: 05.02.2025
(62) Document number(s) of the earlier application(s) in accordance with Art. 76 EPC: 21728438.9 / 4 146 095
(73) Proprietor: Versono Medical Limited, Galway, H91 HP4F (IE)
(72) Inventor: DOLAN, Finbar, Galway, H91 HP4F (IE); O'DONOGHUE, Hugh, Galway, H91 HP4F (IE); TARPEY, Brian, Galway, H91 HP4F (IE); CORBY, Alan, Galway, H91 HP4F (IE); GUILFOYLE, Dion, Galway, H91 HP4F (IE); KINSELLA, Ben, Galway, H91 HP4F (IE); CONNOLLY, Pat, Galway, H91 HP4F (IE); MOONEY, Ivan, Galway, H91 HP4F (IE); O'DONOGHUE, Mary, Galway, H91 HP4F (IE); KANE, Maire, Galway, H91 HP4F (IE); SMEDLEY, Jim, Galway, H91 HP4F (IE)
(74) Representative: Keltie Ltd

(56) References cited:
- WO-A1-2016/081026
- WO-A1-93/16646
- US-A- 5 427 118
- US-A- 5 971 949
- US-A- 6 007 514
- US-A1- 2018 140 321
- US-A1- 2019 262 016
- US-B2- 6 695 782

## Description

This invention relates to treatment of ischaemia by using an ultrasonically-activated wire to cross through a blockage in a blood vessel and, optionally, to facilitate the introduction of follow-on therapeutic devices.

The invention develops concepts expressed in our International Patent Application Nos. PCT/EP2019/080449 (published as WO 2020/094747), PCT/EP2020/081386 (published as WO2021/089847) and PCT/EP2020/081399 (published as WO2021/089859).

As explained in those applications, ischaemia is an inadequate supply of blood to an organ of the body. In atherosclerotic blood vessels, ischaemia occurs as a result of the blood vessels being blocked by obstructions that arise from lesions in the vessel wall, atherosclerotic plaque, or from emboli arising from other sources. Atherosclerotic plaque is composed of materials whose constitution becomes progressively stiffer over time. By partially or fully occluding a blood vessel, a blockage restricts blood flowing to tissues, distal to the blockage, causing cell death and a rapid deterioration in the health of the tissue.

A preferential way to treat such blockages is by minimally invasive, endovascular angioplasties. In these procedures, small-diameter therapeutic devices are introduced into the vasculature and navigated to the blockage via the lumen of veins and arteries, and deployed at the site of the lesion to restore patency. These procedures to revascularise occlusions in the coronary and peripheral arteries in treating chronic atherosclerotic plaques can also be used in the treatment of acute embolic occlusions, thrombi, occlusive blood clots or chronic total occlusions (CTOs).

The anatomies where these procedures are conducted include, but are not limited to, the coronary, neurovascular and the peripheral arteries, including those that service the lower limbs. The different anatomies are associated with different lesions. Lesions found in the various peripheral vessels pose different types of challenges to those found in the coronary arteries. The iliac, femoral, popliteal and infra-popliteal arteries possess varying tortuosity, often substantially less than the coronary or neuro vasculature. However, these arteries are susceptible to extensive calcification which poses a severe impediment to successful endovascular procedures.

In endovascular procedures, an artery is selected and recruited for use in obtaining access to the vasculature. The selection is based on the artery's ability to accommodate the passage of the intended diagnostic or therapeutic device to the target site and the extent to which it may minimise tissue and patient trauma.

In revascularising procedures for peripheral arteries, access is often made by surgical cutdown and puncture to the femoral, popliteal and pedal arteries, commonly known in medical terms as the Seldinger technique. Once the access is made, an introducer wire and an introducer sheath are inserted into the vessel and secured at the site. This sheath acts as a port for the introduction, withdrawal and exchange of devices and to minimise abrasion of the arterial tissue. Then guide catheters and guidewires are introduced into the artery, to provide further protection and to assist device navigation to the target site.

Guidewires are pushed along the lumen of the vessel, carefully to avoid causing any trauma to the vessel wall and are navigated to the site of the obstruction. In successful procedures, the guidewires are then pushed across, or through, the obstruction and are kept in situ to act as a guide over which the diagnostic or therapeutic devices, such as balloon catheters and stents, are tracked to the site of the occlusion.

Guidewires are used in other minimally-invasive procedures to introduce other devices and instruments into vessels or other cavities of the body to enable inspection, diagnosis and different types of treatment. Guidewires are, for example, used for balloon angioplasty, gastrointestinal, urological, and gynaecological procedures. All such procedures require a passageway to be formed through a blockage to facilitate the passage of larger diagnostic or therapeutic devices to the site of lesions or other tissues targeted distal to the lesions in the body.

Visualisation of the progression of guidewires and other devices being advanced through the anatomy is typically done by X-ray or duplex ultrasound. MRI is increasingly popular in other anatomies.

Guidewires are manufactured from various materials, most typically stainless steels and various alloys, including NiTi (nitinol), with many different designs. As an example, specific tapers may be provided over the length of a wire to produce differential degrees of flexibility along the length of the wire. So, at its distal end, the wire will have sufficient flexibility to conform to the shape of the vessel, and strength to transmit force to the tip ('tip stiffness') or force to cross through the lesion.

The wires are made available in a range of outer diameters associated with the anatomies that they are treating. Wires of the order of 0.010" (about 0.25mm) in diameter are commonly used in neuro-vasculatures, whereas wires with an outside diameter of 0.014" (about 0.36mm) are typically used in coronary applications. Such wires are also used in many peripheral vasculatures, typically in infra-popliteal pedal and tibial anatomies. In accessing and treating diseased larger-diameter and straighter vessels such as the iliac, aortic and thoracic vessels, wires with a typical outside diameter of 0.035" (about 0.89mm) may be used. Wires of 0.018" diameter (about 0.46mm) may be used in the lower leg.

The length of wires used in endovascular procedures also varies depending on the distance over which they are considered likely to operate. As an example, wires typically of 750mm up to 900mm in length are used in many peripheral applications where they may be introduced in femoral or popliteal anatomies, or need to track to and through blockages in ipsilateral iliac femoral popliteal and infra popliteal arteries. Wires that are used in contra-lateral and coronary applications tend to be of the order of 1200mm, 1500mm or 1700mm in length. Indeed, wires that may be tracked contra-laterally may be longer, perhaps of the order of 2000mm to 2250mm, 2500mm or 3000mm or more in length.

In some instances, extension wires may be used to facilitate the deployment of certain therapeutic devices. In this instance the proximal end of the wire may require certain features.

Many conventional endovascular wires are passive mechanical devices with no active components. Passive wires do not transmit any energy other than that applied by the clinician. They are operated by their proximal end being pushed, pulled, and torqued to navigate to the blockage site and are then pushed through or around the blockage. However, in very many instances the occlusions are too challenging for conventional wires to cross through. These passive wires then do not work as guidewires are intended to, or they are limited when trying to cross nearly- or totally-occluded blockages that may also be significantly calcified. In situations where they are tracked around occlusions, e.g. in a sub-intimal situation, such wires are often unsuccessful at re-entering the true lumen.

The present invention relates to the use of ultrasonic vibrations transmitted along wires to cross blockages. Transmission of ultrasonic vibrations along small-diameter catheters and assemblies is disclosed in US 3433226. US 5971949 describes the transmission of ultrasonic energy via waveguides of different configurations and tip geometries. US 5427118 describes an ultrasonic guidewire system but does not discuss in detail proximal geometries of the wire or how it facilitates follow-on devices via over-the-wire methods.

Many current single-transducer systems are not ultrasonically activated guidewires but are instead, ultrasonically activated catheters that contain wire members to agitate and ablate material. US 6855123 and US 4979939 describe such systems. These catheters themselves require a separate passive guidewire to help them to navigate and, as such, are tools to facilitate a separate guidewire crossing a blockage. US 9629643 shows a system with a range of distal tip configurations but all requiring a separate guidewire for access.

These ultrasonically activated devices are directed towards delivering an alternative method of revascularisation and are often described as atherectomy devices, crossing devices or vessel preparation devices. In the art, these devices and recanalisation wire devices enhance revascularisation and provide for, or effect, an atherectomy by debulking the lesion by removing the plaque that forms the lesion.

Ultrasonically-activated catheter and wire systems have been considered in the past as a method of atherectomy and to prepare vessels for angioplasty treatment. Some products have been made available commercially in the past, some remain available on the market and some new systems have come to market recently. Such catheter and wire systems often include an ultrasonic generator and an ultrasonic transducer. The ultrasonic generator converts electricity into an ultrasonic waveform, defined by its voltage amplitude, current and frequency. The ultrasonic transducer, and often an amplifying horn, convert the electrical energy into high-frequency mechanical vibrations, defined by frequency and amplitude of vibration.

A small-diameter wire or waveguide serving as a wave delivery system, ultrasound delivery system or transmission member is coupled directly to the transducer, or via any horn, and transmits the mechanical vibrations to the distal tip of the wire. This results in the distal tip of the wire waveguide vibrating at a desired amplitude and frequency with the goal of excavating material and ultimately facilitating the revascularisation or recanalisation of vessels and anatomical structures throughout the body. Tissue and material in the vicinity of the distal tip are affected by a combination of the ultrasonic movement of the tip and its direct mechanical abrasion, ablation and cavitation from the pressure wave components and acoustic streaming that removes ablated material from the zone around the tip.

US2019262016, WO2016081026, US5427118, US6695782, WO9316646, US2018140321 disclose endovascular apparatuses for crossing through an obstruction in a blood vessel.

In general terms, devices of the disclosure employ a combination of a handheld activation unit and an endovascular wire for the purpose of transmitting ultrasonic vibrations to the distal tip of the wire, with the goal of crossing blockages obstructing blood flow, such as chronic total occlusions and other complex lesions. The activation unit houses an ultrasonic transducer and a coupling collet that has a central lumen through the transducer and collet to allow for the passage of the endovascular wire. In such a configuration, the activation unit can slide over the endovascular wire and can be coupled together at a plurality of locations along the wire.

When coupled to the wire, the activation unit transmits ultrasonic energy through the wire and following that can be deactivated, moved to another location on the wire, coupled and reactivated. Following that, the activation unit can be detached from the wire and removed completely, for example by sliding proximally over the wire, while the wire remains in situ, allowing for follow-on procedures. However, the device can also be used in a stand-alone procedure to effect revascularisation and to restore blood flow in pedal applications or other instances.

In known ultrasonically-activated endovascular wire or catheter systems, the proximal end of the guide wire is connected to the transducer. In our patent application published as WO 2020/094747, the wire runs through the transducer and not only extends distally but also proximally from the transducer. This allows the user to couple the transducer to the wire at any desired position and to adjust the total length of the distal portion of the wire from transducer to distal tip, without having to cut the wire.

An adjustable total length of the distal portion of the wire can be very useful for practical purposes, for example to adapt to the expected length of the trajectory the wire tip needs to travel within the patient's body. Also, control of the wire is enhanced in keeping its placement in situ in the vascular lumen whilst adjusting or reconnecting an activation source. Additionally, an adjustable-length distal portion of the wire helps for achieving and optimising resonance at the distal tip at any desired frequency.

When using ultrasonic energy to excite the wire, it is desirable to maximise displacement amplitude at the distal tip of the wire to excavate a lesion. It is also desirable to minimise displacement or movement of a proximal portion of the wire that remains outside the patient's body, both extending both distally and proximally from the activation unit.

In one sense, the invention resides in an endovascular apparatus for crossing through an obstruction in a blood vessel as defined in claim 1. Embodiments of the invention are recited in the dependent claims.

The apparatus comprises: an elongate endovascular element such as a wire, the element comprising a proximal section, a distal tip section of smaller diameter than the proximal section; and a distally-tapering intermediate section extending between the proximal and distal tip sections; an ultrasonic transducer, mechanically coupled to the proximal section of the element for ultrasonically activating the element, hence exciting the distal tip section to facilitate crossing through the obstruction; and a catheter surrounding the element, at least part of the distal tip section of the element protruding distally beyond a distal end of the catheter.

The catheter may be movable longitudinally relative to the activated element to adjust a protruding length of the distal tip section. The catheter may also have a lumen of adjustable diameter defining a variable radial clearance around the element. For example, the catheter may comprise an internal distal collar that is extensible in a radially-inward direction toward the element, such as an annular internal balloon.

The catheter may also comprise an external distal centring arrangement that is extensible from at least two opposed sides of the catheter in radially-outward directions. This may also employ at least one external balloon. Similarly, the catheter may have an external steering arrangement that is extensible from the catheter in at least one radially-outward direction to deflect a distal end of the catheter and the element laterally from a longitudinal axis.

The catheter may contain at least one radially self-expanding cage-like stent or support that is deployable from the catheter around the element and that, when deployed, has a longitudinally-tapering shape.

A proximal end of the catheter may be coupled to the transducer to receive ultrasonic energy from the transducer. For example, the catheter may be coupled to the transducer on a transmission path bypassing a collet that couples the element to the transducer. Alternatively, the catheter may be coupled to the transducer on a transmission path extending through a collet that also couples the element to the transducer. One or more waveguides may extend along the catheter.

Conveniently, the catheter may comprise a longitudinal slit extending along at least a majority of the length of the catheter, the slit communicating with a lumen of the catheter for accommodating the element. Such a slit may terminate short of a distal end of the catheter.

The catheter may define at least two parallel lumens, a first of those lumens being for housing the element and a second of those lumens communicating with a coupling for connection to a pump for driving fluid flow along, or for pressuring fluid within, that second lumen. The second lumen may be in fluid communication with the first lumen.

Correspondingly, the inventive concept encompasses an endovascular apparatus for crossing through an obstruction in a blood vessel that comprises: an elongate endovascular element such as a wire, the element comprising a proximal section, a distal tip section of smaller diameter than the proximal section; and a distally-tapering intermediate section extending between the proximal and distal tip sections; an ultrasonic transducer, mechanically coupled to the proximal section of the element for ultrasonically activating the element, hence exciting the distal tip section to facilitate crossing through the obstruction; and a tube surrounding the element, the tube extending distally from an activation unit that houses the transducer.

At least part of the tube has a structure with greater lateral than longitudinal rigidity. For example, the tube may be extensible and collapsible longitudinally. Conversely, part of the tube may have a structure with greater longitudinal than lateral rigidity. For example, that part of the tube may be user-compressible in a radially-inward direction into contact with the element.

The present disclosure also embraces methods of crossing through an obstruction in a blood vessel, which are not part of the claimed invention.

In one expression, the method involves activating a distal tip section of the wire with ultrasonic energy, causing the distal tip section to move in a primary longitudinal mode with distal and proximal oscillation, and also in a radial direction with a lateral mode and secondary modes of differential harmonics. In another expression, the method involves exciting a distal tip section of an elongate endovascular element such as a wire by conveying ultrasonic energy from a wider proximal section of the element to the distal tip section along a distally-tapering intermediate section of the element, said excitation generating lateral sub-harmonic vibrations in the distal tip section in addition to longitudinal vibrations driven by longitudinal resonances in the element.

Advantageously, the distal tip section undergoes orbital or multi-harmonic movement about a central longitudinal axis to excavate a tunnel in the obstruction that is substantially wider than the distal tip section. For example, the distal tip section of the element may be advanced distally through the obstruction to create an aperture, followed by inducing orbital and/or multi-harmonic movement in the distal tip section on a distal side of the obstruction, and moving the orbitally- or multi-harmonically- moving distal tip section proximally through the obstruction to widen the aperture.

The element may be supported in a surrounding catheter, leaving at least part of the distal tip section protruding distally beyond a distal end of the catheter. Relative longitudinal movement between the element and the catheter allows the catheter to induce orbital and/or multi-harmonic movement in the distal tip section on a proximal side of the obstruction, followed by moving the orbitally- or multi-harmonically- moving distal tip section distally through the obstruction to create or to widen an aperture through the obstruction.

Elegantly, where centring forces are applied by at least one inflatable balloon, the method may comprising: inflating the balloon to centre the catheter before using the element to cross through the obstruction; advancing the balloon into an aperture in the obstruction; and reinflating the balloon within the aperture.

The present disclosure extends to various methods of handling an elongate endovascular element, such as a wire, for crossing through an obstruction in a blood vessel of a patient, which are not part of the claimed invention.

One such method comprises: advancing an activation unit distally toward the patient's body, the activation unit being coupled to the element to activate the element ultrasonically; and by virtue of a tube that extends distally from the activation unit and surrounds a portion of the element, limiting distal advance of the activation unit toward the body to keep that portion of the element outside the body.

Another such method comprises: supporting a portion of the element outside the patient's body within a tube extending distally from an activation unit that is coupled to the element to activate the element ultrasonically; and pressing part of the tube in a radially-inward direction into contact with the element. Conveniently, the tube may be detached from the activation unit while continuing to grip the element by pressing in that part of the tube.

Another such method comprises: supporting a portion of the element outside the patient's body within a tube extending distally from an activation unit that is coupled to the element to activate the element ultrasonically; and varying the length of the tube by applying longitudinal force to a telescopic, concertina or braid structure of the tube.

Another such method comprises: supporting a portion of the element outside the patient's body within a tube extending between the body and an activation unit that is coupled to the element to activate the element ultrasonically, a wall of the tube being penetrated by a longitudinal slit and either: preliminarily, inserting at least part of that portion of the element into the tube through the slit; or subsequently, withdrawing at least part of that portion of the element from the tube through the slit. Where the slit terminates short of a distal end of the tube, the element may be constrained within a distal portion of the tube.

Where the element is supported in a surrounding first lumen of a catheter, fluid may be driven from a second lumen of the catheter into the first lumen around the element. In another approach, fluid may be driven along a second lumen of the catheter to aspirate debris removed from the obstruction by excitation of the distal tip section of the element.

As a result of the ultrasonic activation, movement in the distal tapered region of the wire is composed of both axial and radial or lateral components, the axial component driven by the longitudinal displacement in the transducer, at the drive frequency, while the radial component is composed of a series of lateral modes whose magnitude and shape (amplitude and frequency) is determined by the geometry and flexibility of the wire, the dimensions of the transition region and the geometry of the distal section of the wire.

These wire design features that determine its flexibility, and which allow it to express lateral displacement are selected to encourage coupling at subharmonic frequencies of the driving frequency. As a result, the geometry of the wire in the distal region can be optimised to excavate, abrade or ablate, primarily, in the axial direction or to have a more pronounced lateral movement in addition to the axial or longitudinal movement. The use of both axial and radial movement in the distal region assists in increasing the opening profile during excavation and for subsequent follow-on procedures.

In preferred embodiments, the system of the invention comprises: a signal power generator; an ultrasonic transducer; an optional acoustic horn; a transmitting waveguide or crossing wire, which can transmit high-frequency ultrasonic vibrations to its distal tip to ablate through non-compliant and other materials that are blocking the artery and is of a dimension to facilitate delivery of standard diagnostic and therapeutic devices; and a coupling, being an attachment mechanism that couples the transmission wire to the acoustic horn, or directly to the transducer, that minimises losses and enables the faithful transmission of high-frequency mechanical energy.

The system of the disclosure may comprise a compact housing unit, which may or may not be handheld, to control the operation of the medical device and which houses all or some of the following parts, namely, the signal generator, the ultrasonic transducer, the acoustic amplification horn (although the horn could be part of the transducer assembly or may be omitted) and interface coupling components, as well as data acquisition, processing and system control. In some embodiments, all of these components are aggregated in a single unit. In other embodiments, the components are disaggregated, with the generator housed separately. In another embodiment, the transducer horn is separated. In another embodiment, the coupling connects directly to a transducer stack.

A set, variety or series of interchangeable flexible transmission member assemblies or crossing guidewires are provided for the minimally invasive percutaneous surgical recanalisation of blocked or partially blocked anatomical passageways. A coupling allows the crossing wire to be connected to the ultrasonic transducer and/or horn assembly.

In use: the signal generator provides electrical energy to the transducer; the piezoelectric ultrasonic transducer converts that electrical energy into mechanical vibrations, which may be further amplified by an acoustic horn; the transmission member is coupled to the acoustic transducer or to the horn; the ultrasonic vibrations are transmitted via the transmission member; and the distal tip of the transmission member vibrates at a prescribed frequency and amplitude with the capability of beneficially disrupting the diseased tissue or other material. The ultrasonic transducer may be controlled by a suitable controller to achieve a constant vibration amplitude.

The ultrasonic generator, the main housing, circuitry and coupling components remain external of the patient. Most of the length of the transmission member and components of any peripheral catheter are the only parts of the system that need to enter the patient's body. The proximal section of the transmission member and any peripheral catheter components remain external to facilitate coupling to the main unit and procedural requirements of steering and control.

In the preferred method of operation, not forming part of the claimed invention, an endovascular crossing wire can initially be used in an anatomical passageway in a passive mode with no ultrasonic vibrations. While the wire remains in the anatomical passageway, the crossing wire can then be coupled to the acoustic horn/transducer assembly located in the housing, as required, to energise or transmit ultrasonic vibrations via the wire acting as a transmission member, resulting in vibrations at the distal tip to effect crossing of the lesion. Following ultrasonic activation, the crossing wire can then be detached or decoupled from the acoustic horn located in the housing to return to a passive wire configuration to facilitate further follow-on devices or therapies, if required.

The ultrasonic transducer, horn, coupling means, signal generator, power and control circuitry may all be located in the same hand-portable, lightweight compact housing unit. In another embodiment, the signal generator is separate and is joined to the compact housing unit containing the transducer and horn, via a connector cable.

In another embodiment the entire system may be designed as a single-use device. In another embodiment the ultrasonic transducer, horn, coupling means, generator and control circuitry may all be located in the same portable compact housing unit and connected to power via a cable.

Distal features can be included to enhance performance in navigation and crossing, including control and steerability of the wire optimised for tracking through anatomies and also to increase the opening profile achieved. Additionally, marker bands may be included to provide visibility under fluoroscopy or x-ray. Radio-opaque markers may, for example, indicate the working length and the crossing tip of the wire.

The device may, for example, operate at a set or variable frequency of between 20kHz to 60kHz, preferably of between 35kHz and 45kHz, more preferably of between 37kHz and 43kHz and most preferably around 40kHz. The device may also operate at a desired low power, for example in a range of 1W to 5W or up to 3W, 10W or 15W. In addition to automated control over the desired low-power range of say 1W to 5W, the output of the device can be controlled to allow the user to amplify power beyond this range and so to compensate for unexpected interference and to ensure fast effective crossing. The device can therefore also effect a maximum load at higher power levels, for example up to 50W to 100W to cross challenging lesions assertively, and to overcome attenuation or deflection of the tip.

With the endovascular apparatus according to the invention, it is possible to maximise the displacement amplitude at the distal tip of the wire in an energy-efficient way. For optimal efficiency, it is important that a large portion of the power provided by the transducer is passed on to the distal tip by longitudinal waves through the wire. Any loss of energy by lateral oscillations of the wire is therefore to be minimised. It is also desirable to improve resistance to breakage of the wire.

Damping longitudinal and lateral motion of the proximal portion of the endovascular wire that is outside the patient's body minimises displacement or movement of the proximal portion of the wire. Reducing unwanted motion of the proximal wire portion is important for ensuring the safety of the user and to avoid damaging expensive and sensitive equipment, including the wire itself.

The wire is an example of an elongate endovascular element that may be used as a waveguide or wave delivery system. For example, the element could be a hybrid of a wire and a catheter. In particular, a proximal portion of the element, for example about the first metre of the element from the proximal end, could have a wire encapsulated in a manner akin to a catheter, whereas a distal portion of the element extending to the distal end could be an unencapsulated wire. A wire or other element of the invention could be the inner component of an overall wave delivery system.

In order that the invention may be more readily understood, reference will now be made, by way of example, to the accompanying drawings in which:
Figure 1 is a perspective view of apparatus of the invention;
Figure 2 is a side view of an active wire of the invention;
Figure 3 is a detail side view of an active wire of the invention protruding distally from a catheter;
Figure 4 is a series of drawings showing an active wire excavating a tunnel in a lesion that was blocking a blood vessel;
Figures 5, 6 and 7 are a sequence of views that show an active wire excavating a tunnel in a lesion;
Figure 8 is a side view in longitudinal section of an active wire and catheter coupled to an activation unit;
Figures 9 and 10 show a centring feature of the invention being used for centring to excavate a lesion and subsequently for angioplasty of the lesion after excavation;
Figure 11 shows a steering feature of the invention;
Figure 12 shows a mechanism for varying the lumen of a catheter around an active wire;
Figure 13 shows another wire and catheter arrangement;
Figures 14 and 15 show an arrangement for applying torsion to the catheter to ease its passage through a lesion or obstruction;
Figure 16 shows a variant in which the distal end of the catheter has excavation formations;
Figure 17 shows a variant in which stent-like supports can be deployed from the catheter into the vasculature;
Figure 18 shows a variant in which the wire is accessed through a side window in the catheter;
Figure 19 shows a variant in which the wire and the catheter carry markers;
Figure 20 shows a concept for focusing shockwaves onto a lesion;
Figure 21 shows an enlarged and angled distal end of a catheter;
Figure 22 shows a longitudinally-slit sleeve for receiving and protecting the wire in use;
Figures 23 and 24 show a longitudinally-slit, dual-lumen catheter;
Figure 25 shows the catheter of Figure 24 coupled to a pump for aspiration;
Figure 26 shows further details of a longitudinally-slit catheter;
Figure 27 shows a variant in which the wire is held against an aspiration catheter by a short collar;
Figure 28 shows a variant that provides for fluid communication between adjacent lumens of a catheter;
Figure 29 shows a variant that provides for tactile feedback of relative longitudinal position between a wire and a catheter;
Figure 30 shows a variant in which the catheter has a damping lever for damping excitation of the wire;
Figures 31 and 32 show alternative couplings between the catheter and an activation unit;
Figure 33 shows a distal tube extending from an activation unit around a wire;
Figures 34 to 36 show a distal tube like that of Figure 33 being used to handle the wire and to control its insertion; and
Figures 37 to 39 show further variants of a distal tube.

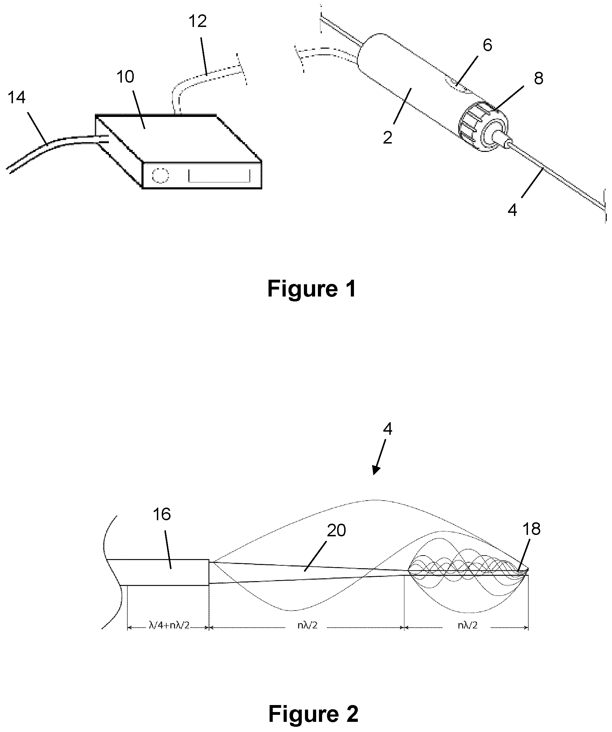

Figure 1 of the drawings shows the overall configuration of a system according to the invention and illustrates some major components of such a system. This example features a handheld ultrasonic activation unit 2 through which a flexible transmission member in the form of an endovascular waveguide or wire 4 extends, in central alignment.

The wire 4 can be inserted into a patient's vasculature and traversed to bring its distal end to the location of a lesion. Once a complex lesion is encountered that resists the wire 4 crossing it, the activation unit 2 can be coupled to the wire 4 at a suitable longitudinal location. When activated, the activation unit 2 transmits ultrasonic vibrations to and along the wire 4, enhancing the ability of the wire 4 to cross the lesion through ablation and other mechanisms. The wire 4 thereby serves as a crossing wire for crossing through an occlusion in a blood vessel and can then remain in situ to serve as a guide wire or rail for delivering subsequent therapeutic devices to treat the lesion.

Typically, the wire 4 may, for example, be more than 2m and up to 3m in length. For example, access to a lesion in or through the foot may involve the wire travelling a distance of typically 1200mm to 2000mm within the vasculature depending on whether an ipsilateral, contralateral or radial approach is chosen. In this respect, a wire 4 tapering distally to a fine wire at its tip can navigate to the pedal arteries and around the pedal arch between the dorsal and plantar arteries. However, the invention is not limited to pedal infra-inguinal or peripheral vessels and could, for example, be used in coronary applications, where the ability of the wire 4 to navigate to and to excavate within tortuous small-diameter arteries is also beneficial.

The diameter of the distal section of the wire 4 will determine the flexibility of that distal section and its ability easily to conform to the shape of the anatomy through which it is intended to pass. Thus, for example, in a tortuous (pedal or coronary) anatomy, a distal section of an appropriate length, of a diameter of, for example, 0.005" (0.127 mm) to 0.007" (0.178 mm) combines appropriate flexibility with the ability to excavate occlusive material for certain Nitinols with particular thermal transition temperatures.

The activation unit 2 may include user controls 6 and optionally also a display. The activation unit 2 further comprises a distal hand toggle 8 that a user can turn about the central longitudinal axis of the unit 2 and of the wire 4. In particular, the activation unit 2 can slide over the wire 4 and can be coupled to the wire 4 at a plurality of longitudinally-spaced locations by applying torque to turn the toggle 8.

To effect coupling, as will be shown in a later drawing, the toggle 8 acts on a collet within the activation unit 2 that surrounds and is coaxial with the wire 4. When the toggle 8 is tightened, the collet grips the wire 4 to transmit ultrasonic energy from an integrated ultrasonic transducer within the activation unit 2, optionally via an amplifier horn that is coupled to the transducer. The wire 4 could be coupled directly to the transducer in some embodiments, in which case the horn may be omitted.

Movement of the toggle 8 is reversible to release the activation unit 2 from the wire 4. Provision is thereby made to interchange wires 4 of different dimensions, configurations or materials for different purposes. There is also the possibility of interchanging the transducer, the horn and/or the collet within the activation unit 2.

In the disaggregated arrangement exemplified in Figure 1, an ultrasonic signal generator 10 is separate from the activation unit 2 and connected to the activation unit 2 by a connector cable 12. Integrated arrangements are also possible in which the ultrasonic signal generator 10 is incorporated into the housing of the activation unit 2.

The example shown in Figure 1 has an externally-powered ultrasonic signal generator 10 and therefore comprises a power cable 14 that connects to an external source of electrical power. Other examples may be powered by internal batteries, which may be incorporated into the ultrasonic signal generator unit 10 or into the activation unit 2.

In general, the components of the system are preferably portable and are more preferably hand-held. The components may be wireless, rechargeable, reusable and recyclable. Any external cable 12, 14 for conveying power or signals may be coupled through a slip ring to allow free rotation of the cable 12, 14 and to avoid entanglement with the wire 4.

When using ultrasonic energy to excite the wire 4, it is desirable to optimise displacement amplitude in the distal tip portion of the wire to excavate and cross a lesion. Conversely, it is desirable to minimise displacement or movement of the proximal end portion of the wire 4, which is outside the patient's body and part of which may hang freely from the proximal side of the activation unit 2. To achieve this, the distal length of the wire 4 from the distal tip to where the activation unit 2 is coupled to the wire 4 should be an odd multiple of a quarter wavelength of the ultrasonic wave.

This creates a standing wave in the wire with a vibrating antinode at the distal tip, hence maximising the amplitude of vibration at the distal tip.

With reference now also to Figure 2, the wire 4 includes regions where the geometry tapers to effect a change in diameter. Specifically, the wire 4 shown in Figure 2 comprises a substantially straight proximal section 16 and a substantially straight distal tip section 18 providing an excavating part for crossing a lesion. The distal section 18 is narrower than the proximal section 16 and can be tapered or can be uniform in diameter along its length.

The distal section 18 is joined to the proximal section 16 by a distally-tapering transition 20. The proximal section 16, the distal section 18 and the transition 20 are in mutual coaxial alignment along a central longitudinal axis of the wire 4, albeit substantially flexible to be bent along their length.

The purpose of the tapered transition 20 is to provide gain and to sustain the transmission of ultrasonic energy through the wire 4. For the purpose of amplification, the change in the cross-sectional area represents a level of gain in both lateral and longitudinal displacement amplitudes in the wire 4. The length and the diameter of the distal section 18 will determine the mode and magnitude of displacement in axial and radial directions. The transition 20 will also affect how a lateral mode of displacement may be established in the distal section 18 of the wire.

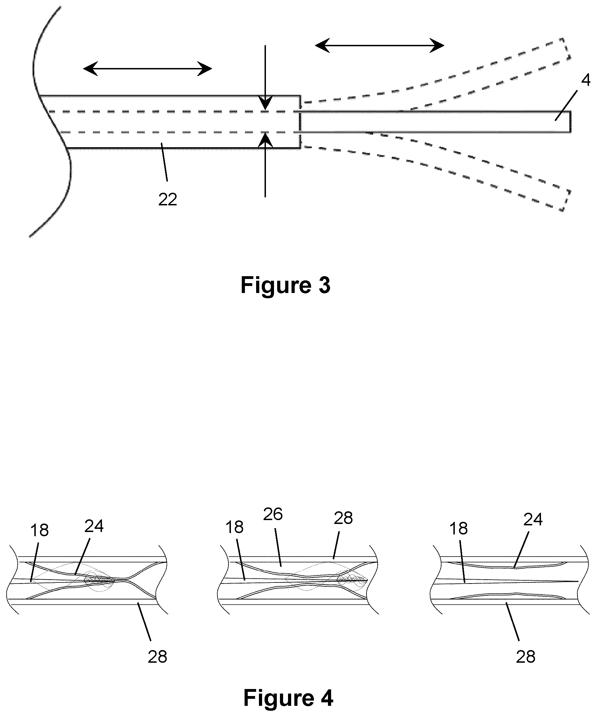

Where the distal section of the wire 4 emerges from a surrounding sheath or catheter 22 as shown in Figure 3 of the drawings, additional lower-frequency lateral vibrations may arise. Freedom of movement enables the lateral component to be expressed and some component of movement may arise from a cantilever effect. In this respect, Figure 3 shows how sleeving the wire 4 in this way leaves a desired distal length free to oscillate laterally as shown. The distal extent of sleeving, and hence the length of the free end of the wire 4, controls excavation by the distal section 18 of the wire 4. Sleeving or jacketing the wire 4 up to a resonant or harmonic length, so that the distal end of the catheter 22 substantially coincides with a resonant or harmonic length, allows the wire 4 to excavate a larger aperture.

Optionally, the catheter 22 and/or the wire 4 can be moved longitudinally relative to each other in distal and proximal directions as shown, for example by turning a thumbwheel on the activation unit 2 that acts on an outer sleeve of the catheter 22 as will be shown in later drawings. As will also be explained, the behaviour of the wire 4 can also be influenced by adjusting radial clearance between the catheter 22 and the wire 4 or by applying radially-inward force from the catheter 22 around the wire 4 as also shown in Figure 3. Squeezing or forceful radial constraint of the wire using a collar such as a balloon has a variable effect depending on the frequency at the time as well as the relative location of the acoustic source and where it is coupled to the wire.

As with all endovascular wires, a balance is required between flexibility expressed as 'trackability' and rigidity expressed as 'pushability' or 'steerability'. Pushability requires longitudinal, columnar stiffness whereas steerability requires torsional stiffness. However, unlike passive wires, the wire 4 must also be able to transmit ultrasonic energy to the distal section 18 in order to assist in crossing lesions. In this way, the wire 4 functions as an excavator, not just at its tip but also along part of its length. In particular, the distal section 18 acts radially as a lateral excavation device for opening an aperture 24 in a lesion 26 within a blood vessel 28 as shown in Figure 4. The wire 4 may also have distal shaped lengths to amplify radial excavation.

As the goal of the activated wire 4 is to cross through the lesion 26 by excavation, its dimensions are optimised with the purpose of excavating as large an aperture 24 as possible at a given input. In this respect, Figure 4 shows how the distal section 18 of the wire 4 may excavate an aperture 24 in the lesion 26 whose diameter is greater than the diameter of the wire 4 and so create a larger lumen through which therapies may be introduced to the lesion 26.

Specifically, the distal section 18 of the wire 4, once activated with ultrasonic energy, moves in a primary longitudinal mode, moving in and out, and also in a radial direction which maps out and excavates a greater volume at the distal end through lateral movement or radial displacement along the wire 4. The distal section 18 of the wire 4 is also seen to move through lateral and undulating movements at or near the drive frequency under the resonant wave and secondary modes of differential harmonics, dependent on the activating frequency, the length of the distal section 18 and the tortuosity of the anatomy. These wave forms may interfere with each other and be more or less effective in excavating material at different moments.

Thus, when activated, the wire 4 acts as an excavation tool that tunnels its way by excavating material distal to the tip 18 of the wire 4 by virtue of longitudinal movement of the wire 4 and then through the offset translation or lateral motion of the wire 4 within the vasculature which provides a lateral offset that opens up the diameter of the tunnel. Thus, the wire 4 abrades the inner surface of the occlusion not just at its distal tip 18 but also along some of its length extending proximally from the distal tip 18 and forms a wider aperture for the passage of follow-up therapeutic devices over the wire 4.

The diameters of the various sections 16, 18, 20 of the wire 4 are chosen for an optimal balance between pushability and trackability, in addition to being able to allow follow-on devices of standard dimensions to use the wire 4 as a guidewire. By way of example, the proximal section 16 may have a diameter of 0.43mm and the distal section 18 may have a diameter of 0.18mm or 0.25mm. The taper in the intermediate transition 20 is slight and so is greatly exaggerated in these drawings. The transition 20 may extend over a multiple of λ in length or a fraction of λ in length, that fraction preferably having with a numerator of 1 and an even denominator - for example in the sequence 1/2, 1/4, 1/8... - whereas the distal section 18 may have a length of λ/2 or a multiple of λ/2 or a fraction of λ/2 such as λ/4. The optimal lengths we have found for the materials being considered for the sections 18 and 20 are λ, λ/2 and potentially λ/4 at lower sub harmonics and for fine wires.

The overall geometry of the wire 4 including its nominal diameter and length and the driving frequency of the system are determined by the characteristic speed of sound in the material of the wire. This characteristic is a function of that material's properties and its geometry. The chosen frequency will produce harmonics along the length of the wire and the loading of the tip of the wire 4 will assist in establishing standing waves. The system may produce lateral and longitudinal displacements over a range of frequencies away from that of the drive frequency, often occurring at sub-harmonics of the frequency in the distal section 18.

In one example, which does not preclude other dimensions, a wire 4 with a core cross section diameter of 0.43 mm defining the proximal section 16 has a tapered transition section 20 optimally located to transition to a distal section 18 of 0.18 mm in diameter. The lengths of each section 16, 18, 20 of the wire 4 can be chosen to have a longitudinal resonant mode at or near the driving frequency, such as 40 kHz, with strong sub-harmonics at or near 20kHz, 10 kHz or others. Through appropriate design, there are neighbouring lateral modes near 40 khz and 20 khz or others. There may be amplification across the taper by a factor of approximately 2.4 or other suitable value.

As a result, through appropriate selection of material, geometry and distal design features, desirable lateral modes will be energised as shown in Figures 2 and 4 even when the wire 4 is driven with longitudinal vibrations. In unison, both the longitudinal and lateral vibrations contribute to excavation of the lesion 26 and result in the wire 4 opening an aperture 24 or lumen in the lesion 26 whose internal diameter is substantially greater than the diameter of the wire 4.

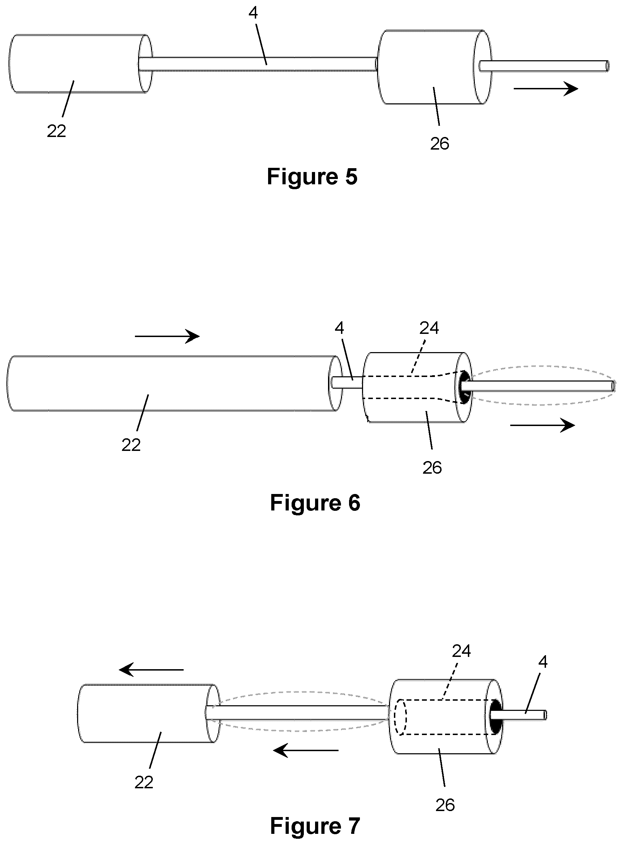

Figures 5, 6 and 7 exemplify how the ability to alter the relative longitudinal position of the wire 4 and the catheter 22 can be exploited to affect the lateral motion of the distal end of the wire 4, thereby influencing secondary or lateral excavation, burrowing or tunnelling of the lesion 26 by the wire 4 within the lesion 26. In particular, Figures 5, 6 and 7 show, schematically, how the distal end of the wire 4 firstly penetrates the lesion 26, as shown in Figure 5, to create a longitudinal aperture 24 and then, with lateral oscillation of the wire 4 optimised, widens the aperture 24 to create a lumen of the desired diameter as shown in Figures 6 and 7.

When a sufficient free end length of the wire 4 extends distally beyond the lesion 26, say greater than approximately λ/4 being about 20mm to 30mm in some examples, lateral oscillation in that free end portion begins lateral excavation of the distal segment of the lesion 26 as shown in Figure 6. At this stage, a longitudinal spacing or clearance of, for example, about 2mm may be maintained between the catheter 22 and the lesion 26 as shown.

Then, drawing the activated wire 4 back proximally through the lesion 26 extends and widens the aperture 24 as shown in Figure 7, aided by lateral oscillation being optimised in the portion of the wire 4 between the catheter 22 and the lesion 26. In this respect, extending the longitudinal spacing between the catheter 22 and the lesion 26 to approximately λ/4 (again about 20mm to 30mm, as one example) maximises lateral oscillation of the wire 4 in the proximal segment of the lesion 26. The free end of the wire 4 may extend distally beyond the lesion 26 by about 2mm, again as an example. If required, the activated wire 4 can then be pushed distally back through the lesion 26 to widen the aperture 24 further.

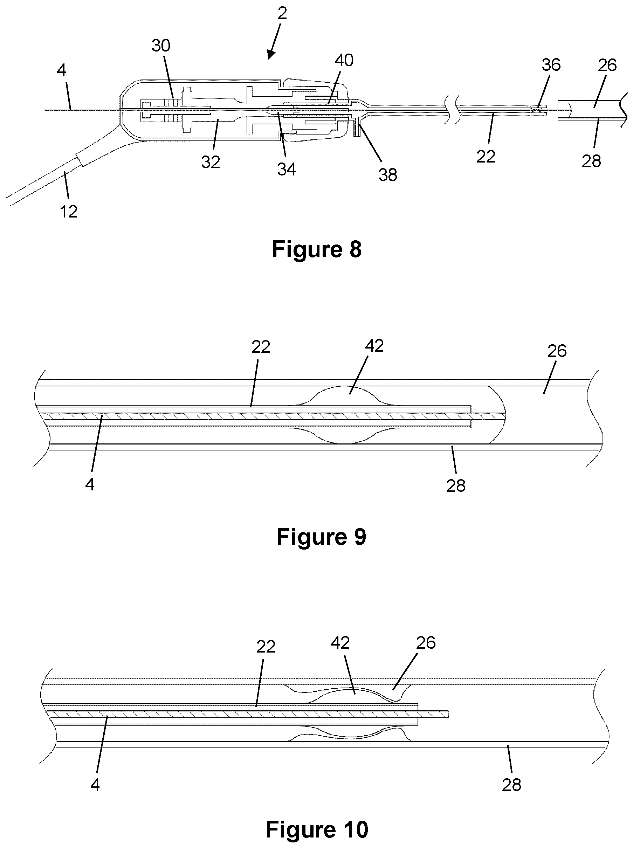

Turning now to Figure 8, this shows an ultrasonic activation unit 2 through which a wire 4 extends longitudinally. In this example, the activation unit 2 is powered externally and is optionally supplied with an ultrasonic signal through a cable 12.

Figure 8 shows that the activation unit 2 contains an ultrasonic transducer 30 and a distally-tapering acoustic horn 32 attached to the distal face of the transducer 30. A collet 34 couples the wire 4 to the distal end of the horn 32.

The transducer 30, the horn 32 and the collet 34 are penetrated by a central lumen to allow for the through-passage of the wire 4. The wire 4 thereby extends though the full length of the activation unit 2 to emerge proximally from the activation unit 2. In other arrangements, the wire 4 could instead emerge from the activation unit 2 laterally at a proximal location with respect to the collet 34.

It will be noted that the activation unit 2 differs from prior art that drives vibration of a wire using an electric motor and a cam or spindle to convert rotary motion to linear motion. Instead, the activation unit 2 uses an ultrasonic transducer 30 that employs the piezoelectric effect of a piezoceramic stack, in which electrical energy is converted into high-frequency axial linear oscillation. Also unlike the prior art, the invention allows the activation unit 2 to be moved along the wire 4 and then to be coupled to transmit ultrasonic energy to the wire 4 at any of a plurality of longitudinally-spaced locations along the wire 4. The active wire 4 performs both longitudinal, axial or directional excavation as well as radial, lateral or orbital excavation through orbiting of the wire 4 out of the axial plane of the wire 4 at different harmonics in a consistent, monotonic manner.

In Figure 8, the catheter 22 that surrounds and supports the wire 4 can be coupled to a distal region of the wire 4. Coupling may be effected via a mechanical coupling but in this example is effected by a distal annular balloon 36 within the catheter 22, which expands into the distal inner lumen of the catheter 22 to narrow the lumen of the catheter 22 and to provide concentric support around the wire 4. This stabilises the wire 4 near the centre of the lumen of the vessel 28 that is blocked by a lesion 26. If the balloon 36 is designed to bear against the wire 4, the surface of the balloon 36 must be able to resist the fretting effect of the ultrasonically active wire 4, for example through a compression ring or other provision that protects the inflated material from being abraded to the point of bursting.

The balloon 36 may be inflated with liquid or gas via an inflation port 38 on the catheter 22 that communicates with the balloon 36 via a channel in the wall of the catheter 22. Additional ports and lumens may be included in the catheter 22, for example to provide for aspiration of embolus or of fragments or particles generated during excavation.

The balloon 36 may be of compliant or non-compliant material. The properties and contact length of the balloon 36 may be tailored to optimise excavation and tunnelling performance, providing sufficient support to the wire 4 while minimising negative damping effects on the wire 4.

Optionally, the balloon 36 or other coupling may be configured to grip the wire 4, applying inward clamping force to the distal portion of the wire 4. In this scenario, ultrasonic energy could be coupled through waveguide elements of the catheter 22, transmitting electromechanical energy from the catheter 22 through to the distal tip region of the wire 4 via the connection through the balloon 36. For this purpose, the balloon 36 could comprise metallic elements or foils to facilitate transmission of energy from the catheter 22 to the wire 4, or other such elements may be interposed between the balloon 36 and the wire 4. The external metallic elements could be in the form of cutting or abrasive elements or could be in the form of a self-expanding cage expanding with the balloon 36. Athertomes could be fixedly attached to the balloon 36.

These features may enhance cutting or crossing performance, and may enhance lateral cutting, particularly when activating the catheter 22. Activating the catheter 22 may also ease its passage through tortuous anatomy.

The proximal end of the catheter 22 may be coupled to the transducer 30 via a mechanical coupling such as an adapter element 40. Later drawings expand on other configurations for coupling between the catheter 22 and the transducer 30 and discuss possible catheter waveguide element configurations. The proximal end of the adapter element 40 abuts the distal end of the horn 32 around the collet 34 and is thereby coupled to the transducer 30 to receive ultrasonic energy.

In principle, the adapter element 40 can facilitate transmission of energy from the transducer in any of three modes of operation, namely: the wire 4 being activated independently; the catheter 22 being activated independently; or the catheter 22 and the wire 4 being activated simultaneously. In the first mode, the adapter element 40 is disengaged from the horn 32, for example by being moved distally away from the horn 32. In the second mode, the collet 34 is disengaged from the horn 32 or from the wire 4, either by being moved distally away from the horn 32 or by being moved radially away from the wire 4.

The adapter element 40 between the transducer 30 and catheter 22 may be tailored to optimise and support the length of wire 4 that would otherwise be left unsupported between the collet 34 and the proximal entrance to the catheter 22. For example, the adapter element 40 may be a variable-length unit, allowing an operator to modify the relative position of the wire 4 and the catheter 22 within a defined operating range. In one example, the adapter element 40 may be a telescopic coupling, providing lateral support to the wire 4 over a larger range of operation. This reduces the risk of breakage of the wire 4 or other negative effects of excessive vibration of the wire 4 outside the catheter 22, such as standing wave formation or damage to the catheter 22.

Figures 9, 10 and 11 show how balloons 42 external to the catheter 22 may be used for various beneficial purposes. Such external balloons 42 can be used individually or in addition to an internal balloon 36 like that shown in Figure 8. For example, in Figures 9 and 10, an external distal annular balloon 42 encircles a distal end portion of the catheter 22.

Figure 9 shows the catheter 22 after being advanced along a vessel 28 close to a lesion 26. The balloon 42 is then inflated, for example via an inflation channel in the wall of the catheter 22. The inflated balloon 42 thereby bears against the surrounding wall of the vessel 38 to centre the catheter 22, and hence the wire 4 that is disposed concentrically within the catheter 22, with respect to the lumen of the vessel 28. When inflated and engaged with the vessel 28 in this way, the balloon 42 also provides support or damping to the wire 4 via the catheter 22 as the wire 4 is subsequently advanced relative to the catheter 22 to cross the lesion 26.

Figure 10 shows an optional next step after the wire 4 has crossed the lesion 26 and has excavated an aperture 24 in the lesion 26 that is wider than the wire 4. Then, the balloon 42 is deflated to enter the lesion 26 and is then reinflated within the lesion 26 in the manner of an angioplasty procedure. Next, the wire 4 may be activated again, hence transmitting ultrasonic energy from the wire 4 through the balloon 42 and into the surrounding lesion 26. The balloon 42 thereby functions to clear a wider cross-sectional area of the lesion 26. However, this is optional as inflation of the balloon 42 alone may itself be helpful to widen the aperture 24 within the lesion 26.

The balloon 42 can be deflated, moved and reinflated two or more times, each time optionally being coupled with the activated wire to clear more of the lesion 26 before follow-up treatment is administered.

If used to transmit ultrasonic energy from the wire 4 into the surrounding lesion 26, the balloon 42 may contain a fluid such as a liquid that acts as a medium when transmitting ultrasonic energy from the wire through the balloon to the lesion. This could improve the effectiveness of energy transmission, acting like an ultrasonic bath. Again, as mentioned above, the balloon 42 may require protective materials to survive abrasion from the activated wire 4, or for a protective layer or element to be interposed between the balloon 42 and the wire 4.

The transmission of mechanical vibration through the balloon 42 may have a beneficial effect on its ability to inflate and to displace plaque in angioplasty.

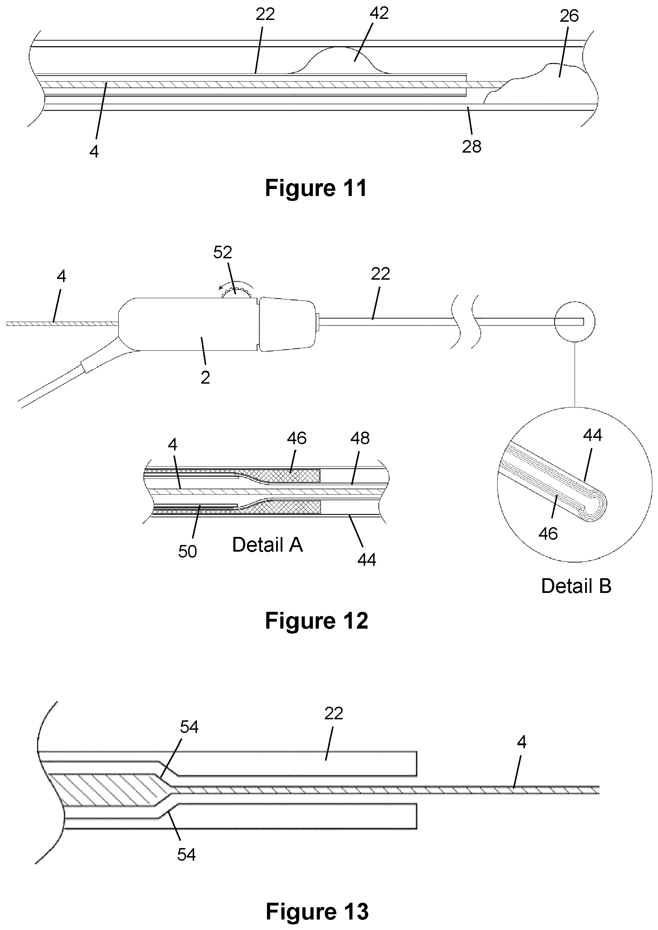

In Figure 11, an external balloon 42 is offset to one side of the catheter 22. When the catheter 22 containing the wire 4 is advanced along a vessel 28 toward a lesion 26, at least a portion of the balloon 42 is inflated to bear against the surrounding vessel 28. The resulting asymmetric force deflects the catheter 22 laterally away from the longitudinal centreline of the vessel 28, hence directing the wire 4 to one side of the centreline. The balloon 42 thereby helps to align the wire 4 with a laterally-offset lesion 26, depending on where the lesion 26 accumulates.

Again, when inflated and engaged with the vessel 28, the balloon 42 provides support or damping to the wire 4 via the catheter 22 as the wire 4 is subsequently advanced relative to the catheter 22 to cross the lesion 26.

It will be apparent that once deflected away from the centreline of the vessel 28, the wire 4 can be steered by turning the catheter 22 about its central longitudinal axis. In this way, the wire 4 can be aimed more accurately at a laterally-offset lesion 26. The wire 4 can also be moved angularly with respect to a large lesion 26 to excavate more of the lesion 26 than might otherwise be possible by remaining aligned with the centreline.

Balloons are not the only way of centring and supporting the wire 4 within the catheter 22. For example, in the arrangement shown in Figure 12, more specifically in Detail A of Figure 12, the catheter 22 comprises multiple layers or tubular components disposed concentrically around the wire 4. Those components are: an outer sheath 44; a radially-compressible body 46 of foam or mesh within the outer sheath 44; an optional isolation layer 48 within the body 46 to isolate the body 46 from the active wire 4; and an inner sheath 50.

The body 46 may, for example, be formed from a shape memory alloy mesh that has been treated to exhibit enhanced elastic properties with a point of inflection in the stress-strain curve on loading.

The inner sheath 50 can be retracted proximally and advanced distally via a user-activated mechanism such as a thumbwheel 52 that may, for example, be located on the housing of the activation unit 2, as shown, or on a proximal hub of the catheter 22.

In a rest state shown in Detail B of Figure 12, the wall of the body 46 expands so that the inner diameter of the body 46 is smaller than the outer diameter of the inner sheath 50. Thus, where the inner sheath 50 is aligned longitudinally with the body 46, the body 46 is deformed radially outwardly by the inner sheath 50.

Specifically, when the inner sheath 50 is advanced distally, it compresses the wall of the body 46 against the inner side of the outer sheath 44. In other words, the inner face of the body 46 is pushed radially outwardly. Conversely, when the inner sheath 50 is retracted proximally by turning the thumbwheel 52, the body 46 is free to expand radially inwardly again, back toward its rest state. In this way, the expanding body 46 narrows the lumen of the catheter 22 supportively around the wire 4 and may bear down on the wire 4.

Advantageously, the porous nature of the body 46 defined by its foam or mesh configuration may help to capture particles generated by ultrasonic breakdown of calcific material of a lesion 26.

Turning now to Figure 13 of the drawings, this shows a catheter 22 with a stepped, distally-tapering inner lumen profile that complements a similarly-profiled wire 4 within. Interlocking between opposed stop formations or shoulders 54 of those profiles prevents further distal movement of the wire 4 beyond a specific point, hence ensuring that only a predetermined length of wire 4 can extend distally beyond the catheter 22. The complementary profiles also help to centre the wire 4 within the catheter 22.

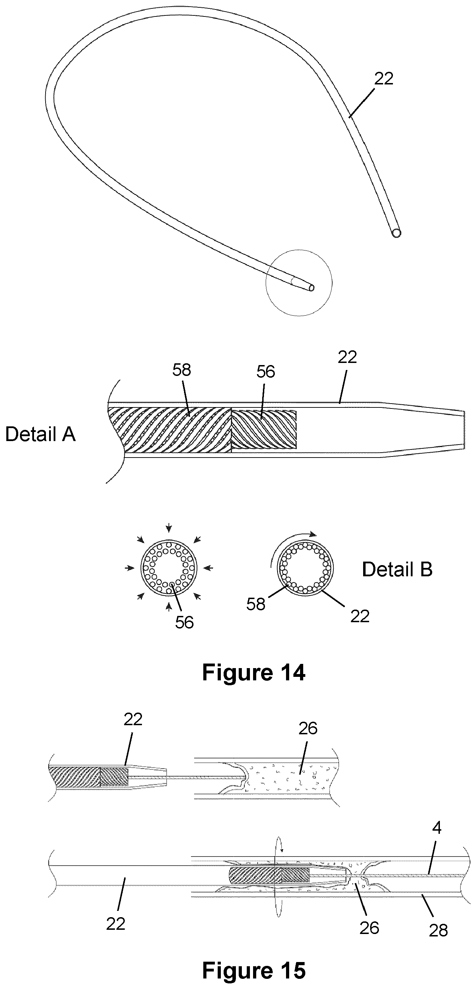

As a catheter 22 is pushed through the inner lumen of a vessel 28 toward a lesion 26 and encounters a lesion 26 or a particularly tortuous path, external pressure on the catheter 22 may cause high static friction, or jamming, that could further increase the difficulty of advancing the catheter 22. Figures 14 and 15 show an embodiment of the invention that addresses this problem.

In this embodiment, the catheter 22 has a distally-tapering end portion and contains two tubular coils of bundled filaments as seen in Detail A of Figure 14, namely an inner coil 56 within an outer coil 58 in telescopic relation. The outer coil 58 is fixed to the interior of the catheter 22. The filaments of each coil 56, 58 follow part-spiral paths so that each coil 56, 58 is twisted about the central longitudinal axis of the catheter 22. The coils 56, 58 are twisted oppositely such that radially-inward movement of the outer coil 58 or proximal movement of the inner coil 56 driven by radially-inward compression of the catheter 22 causes the outer coil 58 to turn about the central longitudinal axis of the catheter 22.

Thus, when the distal end of the catheter 22 encounters a barrier such as a lesion 26 that requires increased distal force to be applied to the catheter 22, this axial force acting on the tapered distal end causes the coils 56, 58 to be pushed together as shown in Detail B of Figure 14 and interact. The interaction between the coils 56, 58 creates a torsional force that causes the distal end of the catheter 22 to rotate, hence generating dynamic friction instead of static friction to ease further distal movement of the catheter 22. For example, the catheter 22 can more easily enter a lesion 26 with rotation about the longitudinal axis, as shown in Figure 15 when following behind a wire 4 that has already crossed through the lesion 26.

The tapered distal tip of the catheter 22 may include one or more metallic elements to increase conduction of ultrasonic energy, and hence excavation of the lesion 26, when the wire 4 is activated and coupled with those elements. The tapered distal tip of the catheter 22 can also include serrated edges or burrs to enhance its cutting performance on encountering the lesion 26.

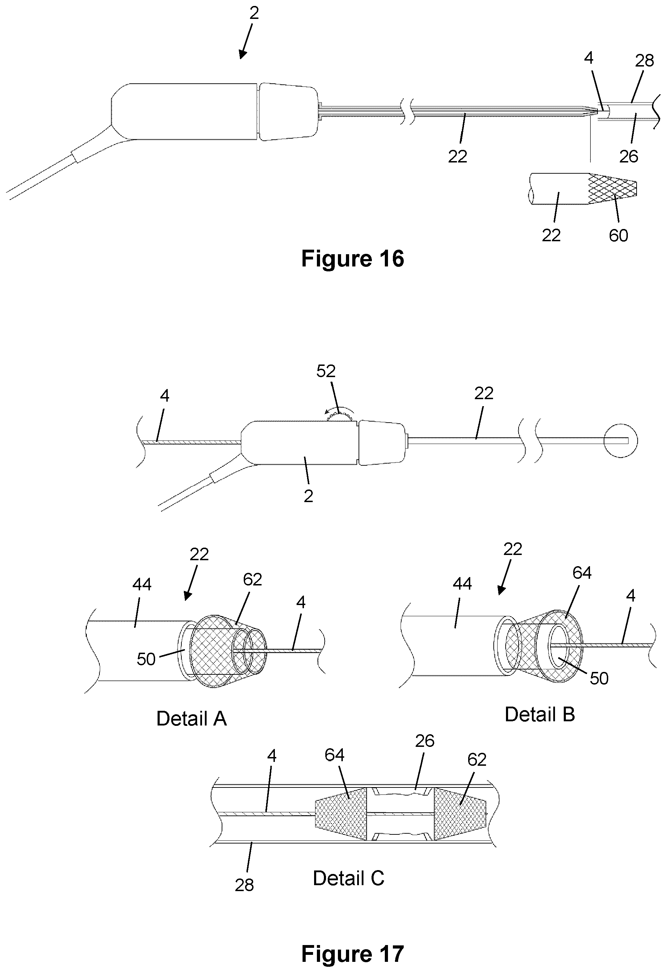

In this respect, Figure 16 shows a catheter 22 with a tapered distal tip including serrated edges, burrs or striations 60 to enhance its tunnelling performance on encountering a lesion 26, shown here blocking a vessel 28. The catheter 22 provides support to a wire 4 advancing into the lesion 26 under ultrasonic activation, specifically providing columnar stiffness for pushability and torsional support for steerability, in addition to providing centring support for the wire 4 within the vessel 28. Synergistically, this interaction and support can enhance the lesion-crossing performance of both the wire 4 and the catheter 22. Supporting the wire 4 and avoiding sharp points or surfaces avoids laceration and promotes abrasion of the lesion 26.

Figure 17 shows another aid that could be used for centring the catheter 22 and hence the wire 4 within the vasculature, namely two variants of an open-cell cage 62, 64 at the distal end of the catheter 22. The cages 62, 64 are mesh or web structures that taper longitudinally around the wire 4 at the distal end of the catheter 22, tapering either in the distal direction as shown for cage 62 in Detail A of Figure 17 or in the proximal direction as shown for cage 64 in Detail B of Figure 17. The cages 62, 64 may taper down to less than the internal diameter of the catheter 22 to centre the wire 4 relative to the catheter 22.

Independently of centring the wire 4, the porous or foraminous structure of the cages 62, 64 may help to capture particles of calcific material generated by ultrasonic breakdown of a lesion 26. The cages 62, 64 may also help with retrieval of clot material after crossing or tunnelling through a lesion 26.

Either of both of the cages 62, 64 are supported on an inner sleeve 50 within the catheter 22. An outer sheath 44 of the catheter 22 can be retracted relative to the inner sleeve 50, for example using a thumbwheel 52 on the activation unit 2, so that the or each cage 62, 64 can be deployed into the vessel 28 as a self-expanding stent.

Only one of the cages 62, 64 can be loaded into the catheter 22 or both of them can be loaded into the catheter 22. Where both cages 62, 64 are loaded into the catheter 22, the distally-tapering cage 62 can be released first and the proximally-tapering cage 64 can be released next with further retraction of the outer sheath 44. For example, as shown in Detail C, the distally-tapering cage 62 can be placed on the distal side of a lesion 26 and the proximally-tapering cage 64 can be placed on the proximal side of a lesion 26 to capture calcific or clot material.

The catheter 22 could be used to aspirate larger quantities of material into the catheter 22 when the cage 62, 64 is expanded, especially in the case of the proximally-tapering cage 64 shown in Detail B of Figure 17. If a cage 62, 64 is closed by a covering, it can also stop or slow blood flow up to an occlusion such as a lesion 26.

Optionally, each cage 62, 64 could have anchoring points or formations, for example on its wider end, for adherence to the fibrous part of a clot or to the vasculature, but without risking penetration of the vasculature.

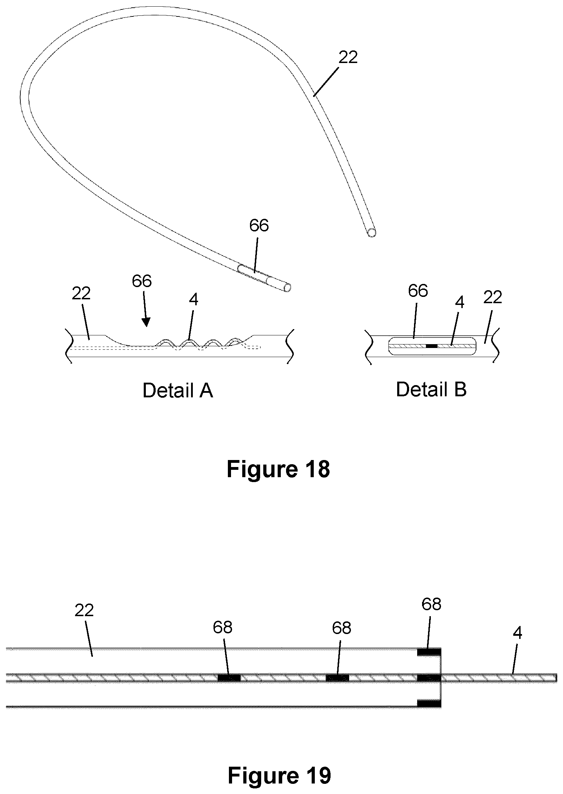

Figure 18 shows another adaptation of the catheter 22, in this example an opening 66 such as a window or a slot in a side of the catheter 22 near its distal end. The laterally-facing opening 66 exposes the active wire 4 to a lesion 26 or to the wall of a vessel 28 from a transverse or sideways direction. This allows the lesion 26 or the wall of the vessel 28 to be targeted with lateral displacement of the wire 4 to enable sideways, lateral or directional atherectomy or debulking. The wire 4 could be allowed to exit the opening 66 in a loop to come into greater contact with the vessel wall.

In this example, as best appreciated in Detail A of Figure 18, the portion of the wire 4 aligned with the opening 66 has an undulating profile defining lateral projections that can protrude through the opening 66 or that can better engage with a lesion 26 disposed adjacent to the window. Detail B of Figure 18 also shows how the wire 4 within the catheter 22 is exposed by the aperture or opening 66.

Figure 19 is a schematic view that shows radio-opaque markers 68 at the distal end of both the wire 4 and catheter 22. When aligned in a specific way, these markers 68 indicate to the operator where to clamp the wire 4 for maximum resonance. This avoids the need for markers at the proximal end of the apparatus but may require the collet 34 of the actuator unit 2 to be locked at a fixed distance from the proximal end of the catheter 22.

Longitudinal vibration of a wire 4 at the distal end of a catheter 22 creates a pressure wave within the fluid that fills the vessel 28, namely blood. At ultrasonic frequencies, those pressure waves can cause cavitation, which beneficially has the potential to erode solid material such as that of a lesion 26 like a chronic total occlusion or CTO. However, the pressure waves propagate in all directions, with random reflections off the surrounding surfaces, in particular the cap at one end of the CTO lesion 26 and the cylindrical wall of the vessel 28. In the consequent absence of a reinforced, standing wave pattern, the energy of cavitation is dissipated.

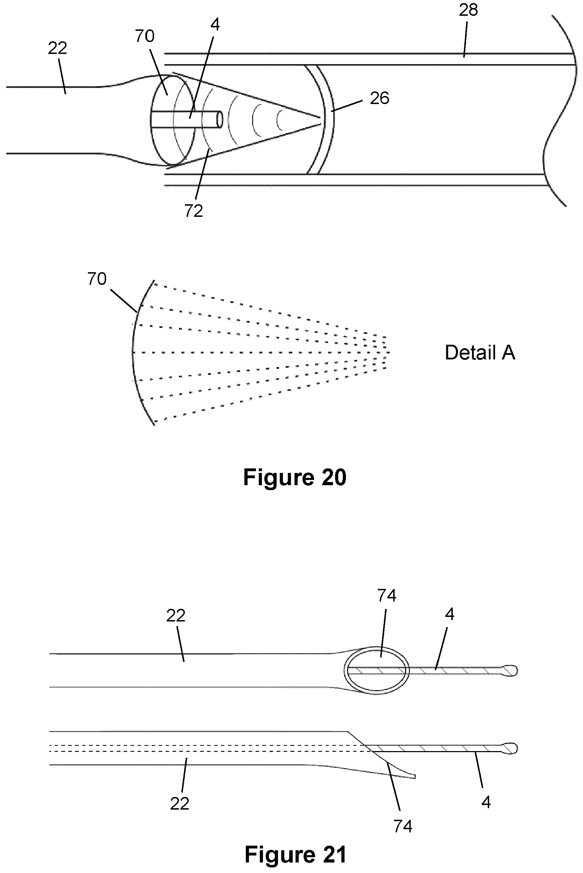

Turning now to Figure 20, this shows a deformable radially-enlarged distal face 70 of the catheter 22. If the distal face 70 of the catheter 22 forms a parabolic surface, and if the diameter of that face 70 is large enough substantially to fill the cross-section of the vessel 28, then pressure waves 72 in fluid within the vessel 28 will be directed to the focal point of the parabola. In this way, energy of the waves 72 may be concentrated onto the lesion 26, where the cavitation will erode the calcified material of a CTO. Such erosion due to cavitation will augment erosion due to the mechanical action of the activated wire 4.

Detail A is a cross-section showing the parabolic shape of the distal face 70 of the catheter 22. With increased concave curvature of that surface, the focal length of the parabola will decrease.

Figure 21 shows another catheter 22 with an enlarged distal end or mouth 74 that lies substantially in a plane acutely angled relative to the longitudinal axis of the wire 4. This wider mouth 74 at the end of the catheter 22 better accepts material or debris when aspirating. Radio-opaque material or markers can be fixed to the angled face to enable visualisation of the location or orientation of the mouth 74 at the distal end.

Beneficially, the acutely-inclined plane of the mouth 74 creates a more tapered or sharply pointed distal profile for the catheter 22, resulting in a lower crossing profile and easier penetration. In this case, the angled mouth 74 also provides coverage of one side of the activated wire 4 to prevent damage to the side wall of a vessel 28 when forward drilling or drilling of only the cap of a CTO lesion 26 is required. This is intended to keep the wire 4 within the true lumen or wire travel between the wall of the vessel 28 and the lesion 26.

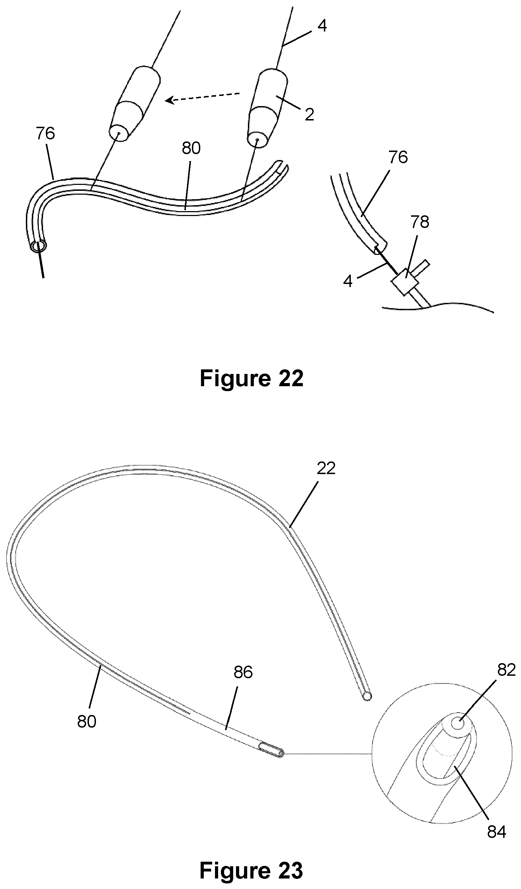

Moving on now to Figure 22, this shows a flexible tubular protective catheter or sheath 76. At the outset of a procedure, the sheath 76 extends along and around the distal portion of the wire 4 extending between the activation unit 2 and the luer or introducer that defines an entry port 78 leading into the patient's body. The purpose of the sheath 76 is to surround and protect this length of wire 4, which would otherwise be exposed before being advanced into the patient's body. The wire 4 can pass easily through the loose sheath 76 while the sheath 76 prevents the wire 4 coming into contact with any materials that could contaminate the wire 4. The catheter or sheath can assist in constraining the lateral component over the length of the catheter or sheath as well as allowing the wire to be immersed in liquid media over its length.

The sheath 76 is particularly useful where a long wire 4 must be inserted into the patient's body, for example to cross a blockage in the distal tibial or pedal arteries. In particular, by providing support or preventing kinking, the sheath 76 helps such a substantial length of wire 4 to be inserted in a single continuous movement rather than intermittently in a succession of shorter movements, as would be the case if the activation unit 2 is instead repeatedly clamped to the wire 4 and released from the wire 4. The sheath 76 could be sized at least partially so that the distal end can be inserted into a support catheter over a portion of its length, for example 20cm.

Thus, instead of being held close to the introducer 78, the activation unit 2 is coupled to the wire 4 at a location remote from the entry port 78 and so is ready for the full length of the wire 4 to be introduced into a target vessel. As a result, the user may simply clamp the activation unit 2 to the wire 4 at a proximal location a metre or more from the patient's body and then can activate and deliver a long section of the wire 4 into the body in a single uninterrupted action. This is useful when activation of the wire 4 may be required to facilitate delivery or tracking of the wire 4.

The sheath 76 must not hinder distal movement of the activation unit 2 and the wire 4 toward the entry port 78. Thus, the sheath 76 may collapse along its length or concertina as the activation unit 2 and the wire 4 are advanced distally. In another approach, as shown here, the sheath 76 has a longitudinal closure or aperture such as a groove or slit 80 along its length to split longitudinally and then peel away from the wire 4. This provides clearance as the activation unit 2 and the wire 4 advance together distally through the sheath 76.

The principle of the longitudinal closure shown in the sheath 76 of Figure 22 can also be applied to catheters, as will now be explained with reference to Figures 23 to 27. These embodiments also disclose multi-lumen catheters 22 comprising at least two parallel lumens. Specifically, these embodiments disclose dual-lumen arrangements comprising: a guidewire lumen 82 to receive and house the ultrasonically-actuated wire 4; and a second lumen 84 to receive an additional or alternative device or for conveying fluid, for example to aspirate embolic materials removed from a lesion during a procedure. Thus, the catheter 22 becomes a dual-purpose aspiration and activation catheter.

The guidewire lumen 82 provides for containment of the wire 4, stabilisation and restraint of lateral displacements of the wire 4 and uniform damping of the wire 4 as it is activated.

The longitudinal closure provides for entry and exit of the wire 4 into or from the guidewire lumen 82 almost anywhere along the length of the catheter 22 and not just at the proximal end. Thus, the wire 4 can enter into or exit from the guidewire lumen 82 at a distal or proximal side-entry point. In particular, the guidewire lumen 82 has a closure such as a slit 80 extending from its proximal end to a distance of about 150mm to 300mm from its distal end. This facilitates the egress and entry of the wire 4 from or into the guidewire lumen 82 as the wire 4 is moved through a vessel and where the user wishes to contain the wire 4 for practical purposes, such as fluid excitation or damping. This feature therefore allows the wire 4 to breach the catheter 22 laterally as it is being advanced through the vasculature and/or any lesion when the wire 4 is ultrasonically actuated.

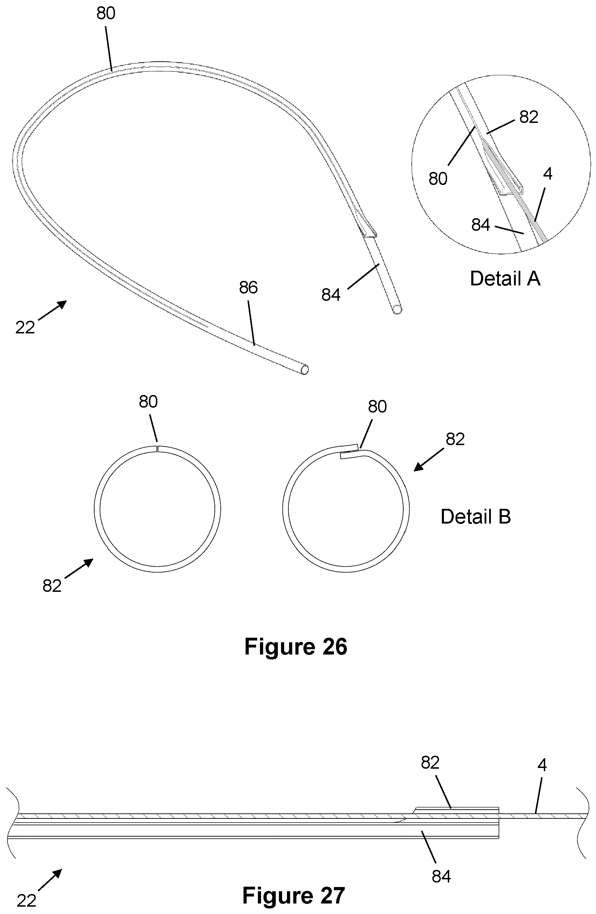

As best appreciated in Detail A of Figure 26, the proximal portion of the guidewire lumen 82 may be defined by a channel section comprising opposed webs divided by the slit 80. Resilience of the channel section pushes the webs together about the slit 80 to encircle and contain the wire 4. The webs may then abut with or overlap each other as shown respectively on the left and on the right in Detail B of Figure 26. Figure 26 also shows that the second lumen 84 may terminate proximally beyond the guidewire lumen 82.

At the slit 80 terminates short of the distal end of the catheter 22, the wall of the guidewire lumen 82 remains intact and circumferentially continuous in that distal portion 86. The integrity of the distal portion 86 enables the wire 4 to guide the catheter 22 along a desired path through the vasculature. The shortness of the distal portion 86 relative to the overall length of the catheter 22 and the wire 4 makes it easier and quicker for a single operator to load and unload the catheter 22 onto the wire 4 during a procedure.

The distal end of the guidewire lumen 82 can be level with the end of the catheter 22 or can be offset proximally from the distal tip of the catheter 22. Conversely, the second lumen 84 is shaped at its distal tip to create an opening with a large area to facilitate the entrapment and aspiration of soft plaque or other embolic materials. For example, the opening can lie in a plane that intersects the second lumen 84 at an acute angle with respect to the central longitudinal axis of the catheter 22.

In another embodiment, the diameter of the second lumen 84 may be greater in a proximal section than in a distal section. Distal tapering of the second lumen 84 may enable it to siphon bulk particles or vulnerable plaque more efficiently.

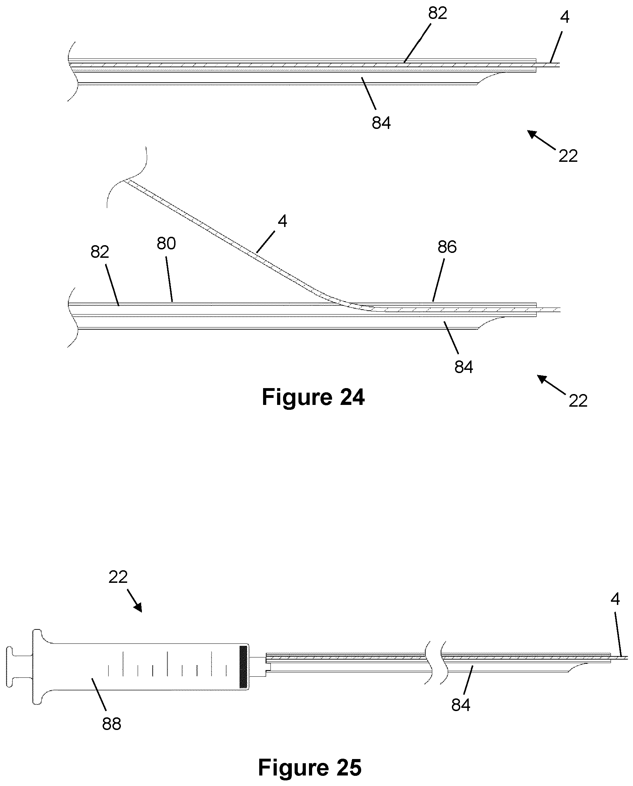

Aspiration is effected through a negative-pressure aspiration pump 88 shown in Figure 25, such as a syringe under vacuum, which is coupled to a proximal port in communication with the second lumen 84. At an appropriate time in the procedure, the aspiration pump 88 can be activated to aspirate and remove any debris that has been created during crossing of a lesion.

As noted above, Figure 26 shows that the second lumen 84 may terminate proximally beyond the guidewire lumen 82. This possibility is taken to the extreme in the variant shown in Figure 27, in which the guidewire lumen 82 is shortened to a collar attached to the distal end region of the second lumen 84 and extending proximally only a short distance from the distal tip of the catheter 22. The wire 4 is only anchored to the catheter 22 by that abbreviated guidewire lumen 82 and is free to splay apart from the second lumen 84 on the proximal side of the guidewire lumen 82. Functionally, the guidewire lumen 82 is akin to the short distal portion 86 beyond the distal end of the slit 80 in Figure 23.

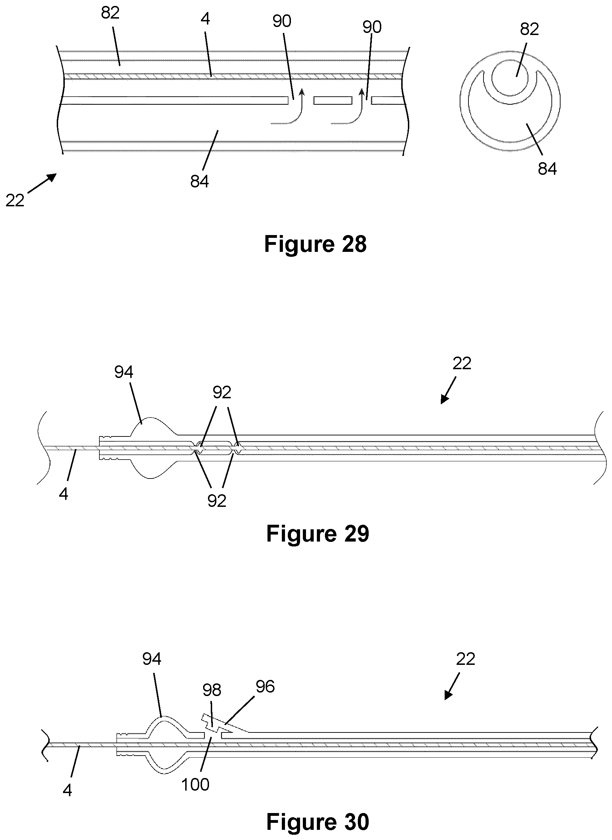

The provision of at least two lumens in the shaft of a catheter 22 can be exploited for other purposes. For example, Figure 28 shows the second lumen 84 used as an infusion lumen and vent holes 90 that penetrate the wall between the second lumen 84 and the guidewire lumen 82. The vent holes 90 serve as cross-channels for the infusion of fluid to enter the guidewire lumen 82 from the second lumen 84, thereby to lubricate motion of the activated wire 4 within the guidewire lumen 82.

The infused fluid could be water or saline solution or a cooling fluid or a form of medication, perhaps to promote lysis.

The fluid-carrying second lumen 84 could be closed at the distal tip so that fluid is delivered through the vent holes 90 at high pressure. This may generate a pressure on the distal end of the wire 4 or may press down on the distal section and affect lateral motion of the wire for some benefit. Distal lateral damping may be effected via a delivered fluid which could have a high or low viscosity or could be a contrast fluid.

Returning to the proximal end of the catheter 22, Figure 29 shows a variant of the catheter 22 that contains internal detent projections 92 facing radially inwardly toward the wire 4. Correspondingly, the wire 4 has complementary projections 92 that face radially outwardly toward the catheter 22. Such projections 92 on the wire 4 could, for example, be defined by bands around the wire 4.

During relative telescopic movement between the catheter 22 and the wire 4 within, the opposed sets of projections 92 ride over each other with a resilient click or snap action. This provides aural and tactile feedback to a user as to the relative longitudinal positions of the catheter 22 and the wire 4 during that telescopic movement.

The catheter 22 shown in Figure 29 also comprises an integral bulbous enlargement 94 near its proximal or hub end. The enlargement 94 provides a convenient rest or stop for the user's fingers to avoid them inadvertently touching the activated wire 4 that may be exposed beyond the proximal end of the catheter 22. In this respect, it will be noted that the projections 92 within the catheter 22 are disposed close to the enlargement 94 on its distal side, hence being aligned with the user's fingers to enhance tactile feedback.

A bulbous proximal enlargement 94 is also a feature of the catheter 22 shown in Figure 30. In this case, the catheter 22 comprises a damping lever 96 close to the enlargement 94 on its distal side, hence again being aligned with the user's grip. The damping lever 96 is resiliently hinged to the wall of the catheter 22 and comprises a stud 98 on its underside that can be received by an opposed opening 100 in the wall of the catheter 22. When the damping lever 96 is depressed, for example by a user's thumb, this pushes the stud 98 through the opening 100 and into contact with an active wire 4 to apply radial damping force to the wire 4. The stud 98 may comprise a braking material that is resistant to abrasion by the wire 4.

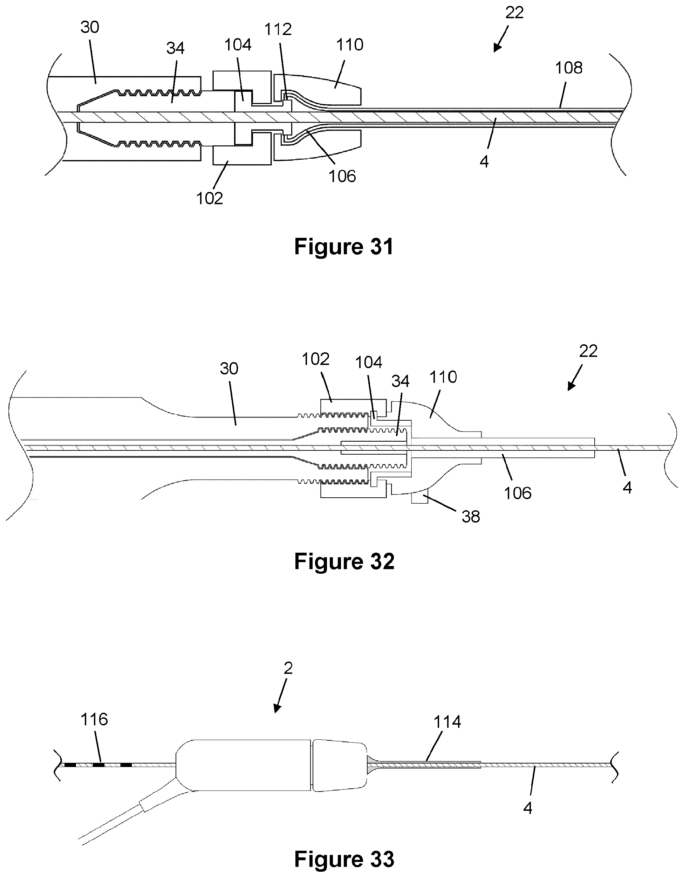

It will be recalled that some aspects of Figure 8 exemplify an interface between the activation unit 2 and the catheter 22. In this respect, Figures 31 and 32 show other arrangements for coupling the catheter 22 to the ultrasonic energy produced by the transducer 30 of the activation unit 2.

In Figure 31, a threaded collet 34 couples the wire 4 to the transducer 30 and a secondary coupling connects the catheter 22 to the collet 34. The secondary coupling comprises: a catheter adaptor cap 102 that has a female thread complementing the male thread of the collet 34; and a catheter coupling ferrule 104 that is received by the catheter adaptor cap 102 and has a through-hole to allow the wire 4 to pass unimpeded through the assembly. A proximal end flange of the coupling ferrule 104 can be compressed against the collet 34 by tightening the adapter cap 102 proximally along its threaded engagement with the collet 34.

The tubular wall of the catheter 22 contains elongate waveguides 106 that extend distally along the length of the catheter 22 and may terminate in a distal mass 108. At their proximal ends where the catheter 22 is received in a hub 110 that can be attached to a housing of the activation unit 2, the waveguides 106 terminate in waveguide terminals 112. The waveguide terminals 112 connect the waveguides 106 to the distal end of the coupling ferrule 104 and hence to the transducer 30 via the coupling ferrule 104. Thus, the transducer 30 is coupled to the wire 4 and to the catheter 22 via the collet 34 to activate the wire 4 and the catheter 22 simultaneously.

The waveguides 106 may be arranged in a straight longitudinal configuration as shown in Figure 31. Alternatively, they may twist helically about the central longitudinal axis of the catheter 22. The catheter 22 may have one or more waveguides 106. Where two or more waveguides 106 are employed, they may extend parallel to each other or may be braided.

In the alternative arrangement shown in Figure 32, the catheter adaptor cap 102 is attached to an externally-threaded distal horn portion of the transducer 30. The adaptor cap 102 presses a proximal flange of the catheter coupling ferrule 104 against the distal end of the transducer 30. Thus, the coupling ferrule 104 is held in direct contact with the transducer mass. For this purpose, the coupling ferrule 104 surrounds the collet 34, which is in separate threaded engagement with the transducer 34. A distal side of the coupling ferrule 104 is coupled to waveguides 106 extending along the catheter 22. Thus, the transducer 30 is coupled to the wire 4 via the collet 34 and to the catheter 22 via the coupling ferrule 94 to activate the wire 4 and the catheter 22 simultaneously.

A proximal hub 110 encloses the coupling mechanism, providing a means of attaching inflation and/or aspiration devices to the catheter 22 via one or more ports 38 on the body of the hub 110.

In the arrangements of Figures 31 and 32, connections may be made to the collet 34 or the transducer 30 using connection means other than mating threads, such as friction taper fits, luer connectors or bayonet connectors.

Turning next to Figure 33, this shows an activation unit 2 with a rigid elongate distally-extending tube 114 forming a tubular nose, sleeve or sheath around the wire 4. The tube 114 protrudes distally from the distal end of the activation unit 2 and surrounds and ensheaths the portion of the wire 4 adjoining the activation unit 2. The tube 114 provides strain relief to the wire 4.

In one mode of use, the tube 114 can be connected to a hub at the proximal end of a catheter. This allows a user to fix the hub to the activation unit 2. In a second mode of use, the tube 114 can fit into the proximal end of a catheter while allowing the hub and the catheter to move telescopically along the tube 114 . In that case, the wire 4 can remain covered as the activation unit 2 and the hub of the catheter are moved closer together or further apart. In both cases, the tube 114 covers the wire 4 protruding from the activation unit 2, protecting the user and the wire 4 itself.

Figure 33 also shows the option of the wire 4 having longitudinally-spaced markers 116. Such markers 116 can guide a user as to the positions at which the activation unit 2 should be coupled to the wire 4 to optimise excavation of a lesion at the distal end of the wire 4.

A tube 114 extending distally from the activation unit 2 around the wire 4 can be used for various purposes in accordance with the invention. For example, complementary projections like those within the catheter of Figure 29 could provide aural and tactile feedback through a click or snap action during telescopic movement between the tube 114 and the wire 4. Similarly, the tube 114 could have a damping lever like that shown on the catheter of Figure 30 whereby a user can apply lateral damping force to the wire 4.

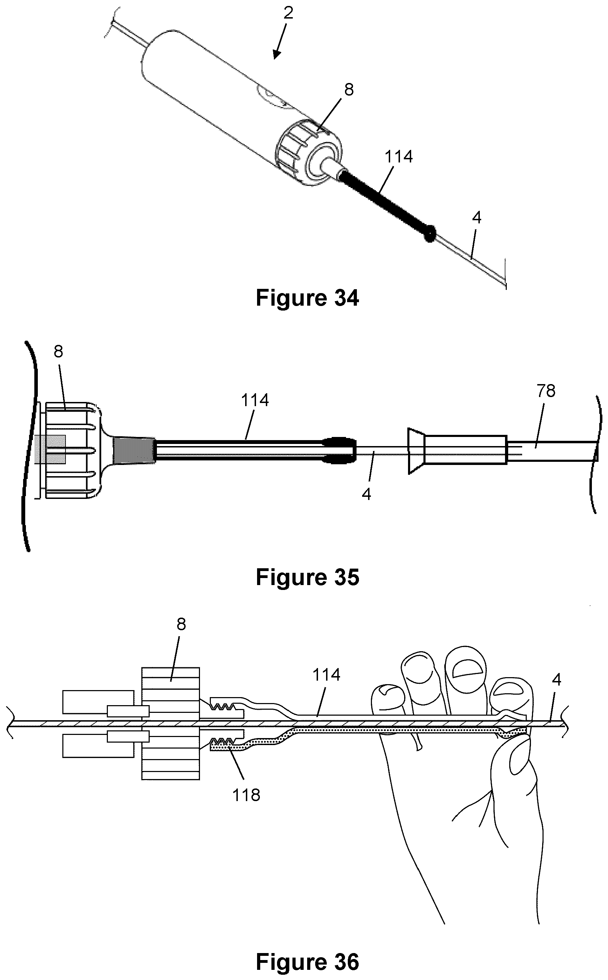

Turning next to Figures 34 to 36, these drawings show a safety feature in which the tube 114 is used to prevent the activation unit 2 being advanced so far distally toward a patient during a procedure that there could be a risk of the wire 4 being lost within the patient's body if the wire 4 breaks.

In particular, as shown in Figure 35, the tube 114 acts as a spacer that prevents a user advancing the activation unit 2 all the way to a luer or introducer that defines an entry port 78 leading into the patient's body. The length of the tube 114 imposes a standoff distance between the activation unit 2 and the entry port 78. The distal end of the tube 114 may be bulbous or otherwise enlarged, as shown, to serve as an end stop or insertion limiter that prevents the tube 114 entering the entry port 78.

The length of the tube 114 ensures that a section of the wire 4 will always remain outside the patient's body even if the wire 4 breaks. Specifically, if the wire 4 fractures near its point of coupling to the transducer 20 within the activation unit 2, a length of wire 4 at least as long as the tube 114, and typically somewhat longer than the tube 114, will always remain outside the entry port 78. In this case, friction between the wire 4 and the tube 114 will help to prevent the wire 4 slipping distally along the tube 114 and into the patient's body.

Figure 36 shows that the tube 114 may have the further attribute of being radially compressible between a user's fingers so as to grip the wire 4 within the tube 114 in the event of breakage. This helps the user to prevent the broken end of the wire 4 being drawn into the patient's body. The tube 114 may be compressible in this way at its proximal end or at any point along its length but is preferably compressible at or near to its distal end as shown.

Advantageously, the tube 114 may also be detached easily from the activation unit 2 to allow quick access to the wire 4. For this purpose, the tube 114 is readily detachable from the distal end of the activation unit 2 by releasing a detachable connector 118 at the proximal end of the tube 114 from the distal side of the toggle 8. There, the tube 114 may, for example, be detached from the activation unit 2 by a twist-off or press/pull arrangement.

Thus, a user can squeeze the tube 114 to grip the fractured wire 4 and then, while still gripping the wire 4, can pull the tube 114 away from the activation unit 2 to ensure that the wire 4 remains outside the patient's body.

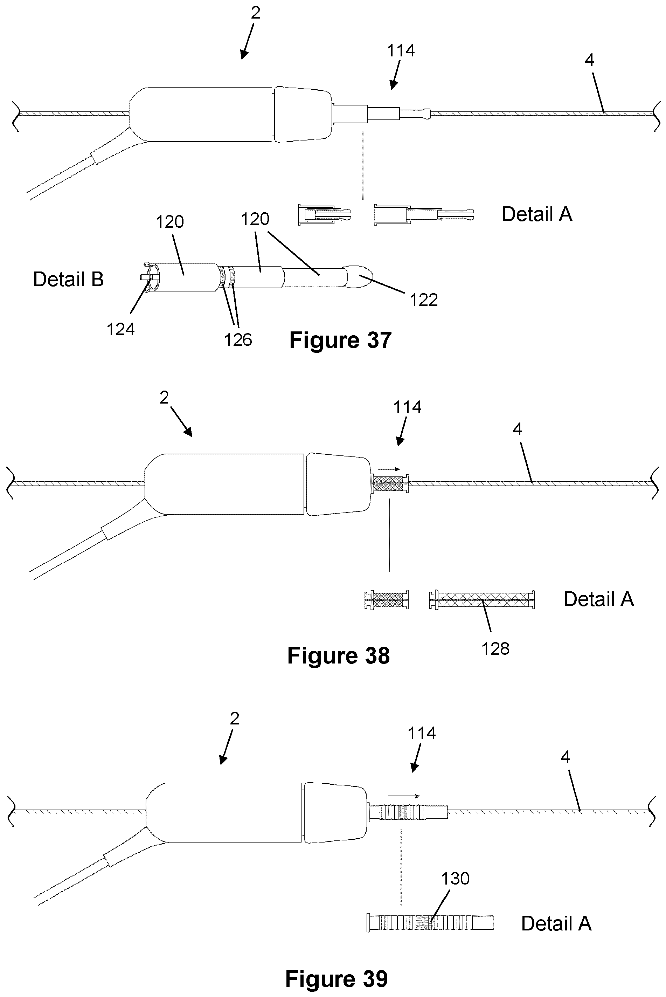

Turning finally to Figures 37 to 39, these drawings show embodiments in which the distal tube 114 is extensible distally and collapsible proximally while remaining attached to the distal end of the activation unit 2.

The provision for longitudinal extension allows the length of the tube 114 to be adjusted to allow for adjustment of damping over different lengths of the wire 4. It also allows the length of the tube 114 to be adjusted to allow for differences in the longitudinal spacing between the activation unit 2 and other structures disposed distally of the activation unit 2, such as the proximal end of a catheter 22 as shown in preceding embodiments, or an entry port 78 as shown in Figure 35. The tube 114 can thereby function as a connector between the activation unit 2 and a structure disposed distally of the activation unit 2.

Thus, the distal tubes 114 shown in Figures 37 to 39 are longitudinally extensible and compressible although they may be rigid, or otherwise less compressible, in a lateral or radial direction so as to protect and guide the wire 4 or to apply damping force to the wire 4 within. In particular, a degree of lateral rigidity may assist with applying damping force to the wire 4.

In Figure 37, the tube 114 comprises a telescopic series of rigid tubular sections 120. The tube 114 is shown in collapsed and extended states in Detail A of Figure 37.

As best shown in Detail B of Figure 37, the distal-most of the sections 120 has a bulbous tip 122 for a user to grip when pulling out the sections 120 to extend the tube 114. A bump-fit feature 124 on the proximal end of the tube 114 allows for easy attachment of the tube 114 to the activation unit 2. Coloured bands 126 in a central section 120 of the tube 114 provide a visual cue so that the user can identify when the tube 114 is at an optimum extension for the wire 4 to perform atherectomy of a lesion at the distal tip of the wire 4.

Figure 38 shows a braided tube 114 that can serve as an ancillary sheath or connection adapter to extend around the wire 4 between the activation unit 2 and the hub of a catheter. A longitudinal slit 128 extends along the full length of the tube 114 to admit the wire 4 laterally into the lumen of the tube 114 if required. Connection of rigid end fittings of the tube 114 to the activation unit 2 and to the catheter hub may be effected by a snap fit, a bump fit or other convenient means.

The braided central portion of the tube 114 allows for longitudinal expansion and contraction. In this respect, the tube 114 is shown in collapsed and extended states in Detail A of Figure 38. Thus, the tube 114 a variable-length unit that allows the operator to modify the relative longitudinal positions of the wire 4 and a catheter within a defined operating range. The extensibility of the tube 114 also provides strain relief or lateral support to the wire 4 over a larger range of operation, reducing the risk of the wire 4 breaking or negative effects of excessive vibration of the wire 4 outside the catheter. Lastly, Figure 39 shows a tube 114 whose extensible central portion has a bellows-like, concertina or accordion construction. A coloured middle section 130 of the tube 114 is revealed as the tube 114 is extended and can be seen fully when the tube 114 is fully extended as shown in Detail A of Figure 39. Again, this provides a visual cue so that the user can identify when the tube 114 is at an optimum extension for the wire 4 to perform atherectomy of a lesion at the distal tip of the wire 4.

Depending upon their characteristics such as rigidity, the tubes 114 shown in Figures 33 to 39 may have various functions. For example, the tubes 114 may provide strain relief to limit the degree of bending that can be applied to the wire 4, noting that NiTi is sensitive to strain and that strain in the region of the collet promotes failure, and by locally constraining the wire 4 against lateral movement. The tubes 114 also enhance safety by contact with the wire 4 so that users can interface with the active wire 4 if they wish.

The tubes 114 can also facilitate capture of the wire as the wire 4 may be wet and slippery with fluid and so may be difficult to grasp, especially when active. A tube 114 can be used to capture a broken wire 4 as noted above but more generally a tube 114 may be used to capture and to hold a wire 4 in place so as not to lose the position of the wire 4, which may be critical to follow-on therapies, and as an aid to optimal positioning of the wire 4 for activation. The tubes 114 also provide a means to enable immersion of the wire 4 in a fluid medium such as a liquid. Longitudinal extensibility of the tube 114 is also useful to keep a contiguous and controlled distance to a port such as a luer lock.

Many other variations are possible within the scope of the appended claims. For example, a multi-lumen catheter could deliver a therapeutic active wire in one lumen alongside or at same time as a passive guidewire in another lumen.

## Claims

1. An endovascular apparatus for crossing through an obstruction (26) in a blood vessel (28), the apparatus comprising:
an elongate endovascular element (4) such as a wire, the element (4) comprising a proximal section (16), a distal tip section (18) of smaller diameter than the proximal section (16); and a distally-tapering intermediate section (20) extending between the proximal and distal tip sections (16, 18);
an ultrasonic transducer (30), mechanically coupled to the proximal section (16) of the element (4) for ultrasonically activating the element (4), hence exciting the distal tip section(18) to facilitate crossing through the obstruction (26); and
a tube (114) surrounding the element (4), **characterised in that** the tube (114) extends distally from
an activation unit (2) that houses the transducer (30), and **in that** part of the tube (114) has a structure with greater longitudinal than lateral rigidity, that part of the tube (114) being user-compressible in a radially-inward direction into contact with the element (4).

2. The apparatus of Claim 1, wherein at least part of the tube (114) has a structure with greater lateral than longitudinal rigidity.

3. The apparatus of Claim 2, wherein the tube (114) is extensible and collapsible longitudinally.

4. The apparatus of Claim 3, wherein the tube (114) comprises a telescopic series of rigid tubular sections (120).

5. The apparatus of Claim 3, wherein the tube (114) comprises a concertina or braid structure.

6. The apparatus of any preceding claim, where the activation unit (2) further comprises a toggle (8) that can be turned to couple the activation unit (2) to the element (4), the apparatus further comprising a connector (118) positioned at a proximal end of the tube (114) and detachable from a distal side of a toggle (8).

7. The apparatus of Claim 6, wherein the connector (118) is operable to detach the tube (114) from the toggle (8) by applying a twist-off or press/pull action to the tube (114).

8. The apparatus of any preceding claim, wherein the tube (114) is connectable to a hub at a proximal end of a catheter (22).

9. The apparatus of Claim 8, wherein the tube (114) fits into the proximal end of the catheter (22) while allowing the hub and the catheter (22) to move telescopically along the tube (114), whereby the element (4) remains covered as the activation unit (2) and the hub of the catheter (22) are moved closer together or further apart.

10. The apparatus of any preceding claim in combination with an entry port (78) such as a luer, wherein a distal end (94) of the tube (114) is bulbous or otherwise enlarged to serve as an insertion limiter that prevents the tube (114) entering the entry port (78).

11. The apparatus of any preceding claim, wherein the tube (114) comprises a damping lever (96) that is resiliently hinged to a wall of the tube (114) and comprises a stud (98) on its underside that can be received by an opposed opening (100) in the wall of the tube (114) when the damping lever (96) is depressed to push the stud (98) through the opening (100) and into contact with the element (4).

## Patentansprüche

1. Eine endovaskuläre Vorrichtung zum Durchqueren einer Obstruktion (26) in einem Blutgefäß (28), wobei die Vorrichtung Folgendes beinhaltet:
ein längliches endovaskuläres Element (4) wie etwa einen Draht, wobei das Element (4) einen proximalen Abschnitt (16), einen distalen Spitzenabschnitt (18) mit einem kleineren Durchmesser als der proximale Abschnitt (16); und einen sich distal verjüngenden Zwischenabschnitt (20) beinhaltet, der sich zwischen dem proximalen und dem distalen Spitzenabschnitt (16, 18) erstreckt;
einen Ultraschallwandler (30), der mechanisch mit dem proximalen Abschnitt (16) des Elements (4) gekoppelt ist, zur Ultraschallaktivierung des Elements (4), wodurch der distale Spitzenabschnitt (18) angeregt wird, um das Durchqueren der Obstruktion (26) zu erleichtern; und
eine Röhre (114), die das Element (4) umgibt, **dadurch gekennzeichnet, dass** sich die Röhre (114) distal von einer Aktivierungseinheit (2) erstreckt, in der der Wandler (30) untergebracht ist, und dadurch, dass dieser Teil der Röhre (114) eine Struktur mit einer größeren longitudinalen als lateralen Steifheit aufweist, wobei dieser Teil der Röhre (114) durch den Benutzer in einer radial nach innen gerichteten Richtung komprimiert werden kann, um in Kontakt mit dem Element (4) zu kommen.

2. Vorrichtung gemäß Anspruch 1, wobei mindestens ein Teil der Röhre (114) eine Struktur mit einer größeren lateralen als longitudinalen Steifheit aufweist.

3. Vorrichtung gemäß Anspruch 2, wobei die Röhre (114) längs ausziehbar und einziehbar ist.

4. Vorrichtung gemäß Anspruch 3, wobei die Röhre (114) eine teleskopische Reihe steifer röhrenförmiger Abschnitte (120) beinhaltet.

5. Vorrichtung gemäß Anspruch 3, wobei die Röhre (114) eine Falt- oder Flechtstruktur beinhaltet.

6. Vorrichtung gemäß einem der vorhergehenden Ansprüche, wobei die Aktivierungseinheit (2) ferner einen Umschalter (8) beinhaltet, der gedreht werden kann, um die Aktivierungseinheit (2) mit dem Element (4) zu koppeln, wobei die Vorrichtung ferner ein Verbindungselement (118) beinhaltet, das an einem proximalen Ende der Röhre (114) positioniert ist und von einer distalen Seite eines Umschalters (8) abnehmbar ist.

7. Vorrichtung gemäß Anspruch 6, wobei das Verbindungselement (118) betriebsfähig ist, um die Röhre (114) durch Anwenden einer Abdreh- oder Drück/Zieh-Bewegung auf die Röhre (114) von dem Umschalter (8) abzunehmen.

8. Vorrichtung gemäß einem der vorhergehenden Ansprüche, wobei die Röhre (114) mit einem Ansatz an einem proximalen Ende eines Katheters (22) verbunden werden kann.

9. Vorrichtung gemäß Anspruch 8, wobei die Röhre (114) in das proximale Ende des Katheters (22) hineinpasst, während sich der Ansatz und der Katheter (22) teleskopisch entlang der Röhre (114) bewegen können, wodurch das Element (4) bedeckt bleibt, während die Aktivierungseinheit (2) und der Ansatz des Katheters (22) enger zusammen oder weiter auseinander bewegt werden.

10. Vorrichtung gemäß einem der vorhergehenden Ansprüche in Kombination mit einem Eintritts-Port (78) wie etwa einem Luer, wobei ein distales Ende (94) der Röhre (114) knollenförmig oder auf andere Weise vergrößert ist, um als ein Einschubbegrenzer zu dienen, der verhindert, dass die Röhre (114) in den Eintritts-Port (78) eintritt.

11. Vorrichtung gemäß einem der vorhergehenden Ansprüche, wobei die Röhre (114) einen Dämpfungshebel (96) beinhaltet, der elastisch schwenkbar an einer Wand der Röhre (114) angebracht ist und einen Zapfen (98) auf seiner Unterseite beinhaltet, der von einer gegenüberliegenden Öffnung (100) in der Wand der Röhre (114) aufgenommen werden kann, wenn der Dämpfungshebel (96) nach unten gedrückt wird, um den Zapfen (98) durch die Öffnung (100) zu drücken und in Kontakt mit dem Element (4) zu bringen.

## Revendications

1. Appareil endovasculaire pour traverser une obstruction (26) dans un vaisseau sanguin (28), l'appareil comprenant :
un élément endovasculaire allongé (4) tel qu'un fil, l'élément (4) comprenant une section proximale (16), une section de pointe distale (18) de diamètre inférieur à celui de la section proximale (16) ; et une section intermédiaire (20) s'amincissant distalement, s'étendant entre la section proximale et la section de pointe distale (16, 18) ;
un transducteur ultrasonore (30), couplé mécaniquement à la section proximale (16) de l'élément (4) pour activer l'élément (4) par ultrasons, excitant ainsi la section de pointe distale (18) pour faciliter la traversée de l'obstruction (26) ; et
un tube (114) entourant l'élément (4), **caractérisé en ce que** le tube (114) s'étend distalement à partir d'une unité d'activation (2) qui loge le transducteur (30), et **en ce qu'**une partie du tube (114) a une structure présentant une rigidité longitudinale supérieure à sa rigidité latérale, cette partie du tube (114) pouvant être comprimée par l'utilisateur dans une direction radialement vers l'intérieur jusqu'à entrer en contact avec l'élément (4).

2. Appareil selon la revendication 1, dans lequel au moins une partie du tube (114) a une structure présentant une rigidité latérale supérieure à sa rigidité longitudinale.

3. Appareil selon la revendication 2, dans lequel le tube (114) est extensible et rétractable longitudinalement.

4. Appareil selon la revendication 3, dans lequel le tube (114) comprend une série télescopique de sections tubulaires rigides (120).

5. Appareil selon la revendication 3, dans lequel le tube (114) comprend une structure en accordéon ou en tresse.

6. Appareil selon l'une quelconque des revendications précédentes, dans lequel l'unité d'activation (2) comprend en outre un bouton (8) qui peut être tourné pour coupler l'unité d'activation (2) à l'élément (4), l'appareil comprenant en outre un connecteur (118) positionné à une extrémité proximale du tube (114) et détachable d'un côté distal d'un bouton (8).

7. Appareil selon la revendication 6, dans lequel le connecteur (118) peut être actionné pour détacher le tube (114) du bouton (8) en appliquant au tube (114) une action de détachement par rotation ou de pression/traction.

8. Appareil selon l'une quelconque des revendications précédentes, dans lequel le tube (114) peut être raccordé à une embase à une extrémité proximale d'un cathéter (22).

9. Appareil selon la revendication 8, dans lequel le tube (114) s'emboîte dans l'extrémité proximale du cathéter (22) tout en permettant à l'embase et au cathéter (22) de se déplacer télescopiquement le long du tube (114), de sorte que l'élément (4) reste couvert lorsque l'unité d'activation (2) et l'embase du cathéter (22) sont rapprochées ou éloignées l'une de l'autre.

10. Appareil selon l'une quelconque des revendications précédentes en combinaison avec un orifice d'entrée (78) tel qu'un raccord Luer, dans lequel une extrémité distale (94) du tube (114) est renflée ou autrement élargie pour servir de limiteur d'insertion qui empêche le tube (114) de pénétrer dans l'orifice d'entrée (78).

11. Appareil selon l'une quelconque des revendications précédentes, dans lequel le tube (114) comprend un levier d'amortissement (96) qui est articulé de manière élastique à une paroi du tube (114) et comprend un ergot (98) sur sa face inférieure qui peut être reçu par une ouverture opposée (100) dans la paroi du tube (114) lorsque le levier d'amortissement (96) est enfoncé pour pousser l'ergot (98) à travers l'ouverture (100) et en contact avec l'élément (4).
